# EUROPEAN PATENT APPLICATION

(11) **EP 4 726 027 A1**
(43) Date of publication of application: **15.04.2026**
(21) Application number: 24819434.2
(22) Date of filing: 07.06.2024
(51) Int. Cl.: C12N 1/21, C12N 1/15, C12N 1/19, C12N 5/10, C12N 15/52, C12P 1/00, C12Q 1/06, C40B 40/02

(54) **PRODUCTION OF SUBSTANCE BY STRAIN**

(30) Priority: 09.06.2023 JP 2023095819
(71) Applicant: Synplogen Co., Ltd., Kobe-shi, Hyogo 650-0047 (JP)
(72) Inventor: MIYAMOTO, Naoki, Kobe-shi, Hyogo 650-0047 (JP); TSUGE, Kenji, Kobe-shi, Hyogo 650-0047 (JP)
(74) Representative: Engelhard, Markus
(86) International application number: PCT/JP2024/020950
(87) International publication number: WO 2024/253207

(57) **Abstract**

The present disclosure provides a method for producing a microbial strain exhibiting a desired property such as high production and suppression of by-products by activating a gene cluster. Specifically, the present disclosure provides a method for producing a strain having a desired property, the method including: a step 1) of providing a plurality of constructs in which promoters are arranged in a monocistronic configuration, the construct including a gene cluster containing a plurality of genes; a step 2) of performing combinatorial library construction for the promoters in the construct and introducing the construct into a desired host cell; and a step 3) of selecting, in the host cell, a strain having a desired property.

## Description

### Technical Field

The present disclosure relates to production of a substance by a strain exhibiting a desired property.

### Background Art

Approximately half of the pharmaceuticals that have been developed and used by humankind to date are natural products or substances derived from natural products (Non Patent Literature 1). To date, it has been presumed that the natural products accessible to humankind had reached a plateau; however, genome sequencing of microorganisms has revealed that a large number of unused natural products still remain in microbial genomes. Most of these are not produced under ordinary laboratory conditions (dormant state) or are undetectable because they are produced only in trace amounts (Non Patent Literature 2). When these biosynthetic gene clusters for the natural products can be forcibly activated to efficiently produce substances, it can be expected that novel substances that have not been obtained to date, or useful substances, can be efficiently obtained.

Among natural products, polyketides (PKs) and non-ribosomal peptides (NRPs) are groups of natural products produced by various microbial species such as actinomycetes, filamentous fungi, and Bacillus subtilis, and these groups include many molecules having useful physiological activities for humankind, such as antibiotic and anticancer activities. However, the molecular structures thereof are extremely complex, and therefore, the majority of them are impossible or difficult to be commercially produced by chemical synthesis. That is, in order to utilize these industrially, a biological production method is required. Gene clusters of polyketide synthases (PKSs) or non-ribosomal peptide synthases (NRPSs) contained in microbial genomes are generally very long chains, and because they contain abundant repetitive sequences and have characteristics such as high GC content or high AT content, they are DNA sequences that are difficult to synthesize by conventional methods.

Therefore, by cloning from the genome of a target microorganism and transferring the cloned fragment onto a desired vector, a construct in which control factors or the like are substituted has been produced, and such constructs have been used for homologous or heterologous production. As an example, cases have been reported in which an increase in production through substitution with a promoter stronger than the wild-type promoter has been achieved by techniques such as homologous recombination in yeast (Non Patent Literature 3) or CRISPR-Cas9 (Non Patent Literature 4 and Patent Literature 1).

However, due to the complexity of the sequences of gene clusters, there are significant constraints on engineering of such sequences. In addition, in many wild-type PKSs or NRPSs, a single promoter generally governs the activation of transcription of a plurality of component genes or the entire gene cluster. In the case of activation of dormant genes or heterologous production, if transcription is controlled in a monocistronic manner on a gene-by-gene basis, and proteins derived from each gene are transcribed and translated at appropriate times during microbial growth, it is presumed to be effective for increasing the production. However, for the reasons described above, such realization has been difficult with conventional techniques.

### Citation List

### Patent Literature

Patent Literature 1: KR102301301B1

Non Patent Literature 1: D. J. Newman, et. al., J. Nat. Prod., 83, 770 (2020)
Non Patent Literature 2: C. C. Brett, et. al., Annu. Rev. Biochem., 90, 763 (2021)
Non Patent Literature 3: Kang H-. S., et. al., ACS Synth. Biol., 5(9), 1002 (2016)
Non Patent Literature 4: Kim H., et. al., ACS Synth. Biol., 9(1), 175 (2020)

### Summary of Invention

### Solution to Problem

In view of the above, the present disclosure has been completed by producing a target substance in a host cell such as Bacillus subtilis through monocistronic transcription of each gene constituting a natural product biosynthetic enzyme gene cluster using growth-phase-dependent promoters with appropriate transcription strengths. That is, the gist of the present disclosure is as follows.

### (Item X1)

A method for producing a strain having a desired property, the method including:
a step 1) of providing a construct including a gene cluster and a plurality of promoters;
a step 2) of performing combinatorial library construction for the promoters in the construct; and
a step 3) of selecting, in a host cell, a strain having a desired property.

### (Item X1-1)

The method according to the preceding item, in which the gene cluster contains a plurality of genes.

### (Item X1-2)

The method according to any one of the preceding items, in which the promoter is operably linked to the gene.

### (Item X1-3)

The method according to any one of the preceding items, in which the step 1) includes a step of providing a construct into which a plurality of promoters are introduced, the construct including a gene cluster containing a plurality of genes.

### (Item X2)

The method according to any one of the preceding items, in which the plurality of promoters are arranged in a monocistronic configuration for each of the genes contained in the gene cluster, are arranged in a polycistronic configuration for all of the genes in the gene cluster, or are arranged in a mixture of a monocistronic configuration and a polycistronic configuration with respect to the genes in the gene cluster.

### (Item X3)

The method according to any one of the preceding items, in which the strain having the desired property has at least one property selected from the following properties: a property of being able to produce a target substance; a property of highly producing a target substance; a property of retaining a target substance within the cells without secreting the target substance; a property of secreting a target substance extracellularly; a property of enabling easy culture accompanied by production of a target substance; a property of being able to produce a plurality of target substances in a desired ratio; a property of producing a target substance without producing harmful substances; a property of having robust production of a target substance; a property in which no deletion occurs in a gene for producing a target substance during a culture period; a property of enabling control of production of a target substance; a property of being able to suppress production of substances other than a target substance; a property of being able to suppress production of by-products and/or impurities that are difficult to separate by affinity, ion exchange, gel filtration, reversed phase, normal phase, chiral chromatography, distillation, or crystallization; a property of producing a small amount of empty capsids in viral vector production; and a property in which no deletion occurs in a plasmid.

### (Item X3-1)

The method according to any one of the preceding items, in which the empty capsid ratio is 80% or less, preferably 70% or less, more preferably 60% or less, 50% or less, 40% or less, 30% or less, 20% or less, or 10% or less, and most preferably 0%.

### (Item X4)

A method including performing an action based on the desired property by using a strain produced by the method according to any one of the preceding items.

### (Item X4-1)

The method according to any one of the preceding items, in which the action includes performing an action corresponding to the following properties:
· a property of being able to produce a target substance: performing an action of producing a target substance;
· a property of highly producing a target substance: performing an action of producing a target substance;
· a property of retaining a target substance within the cells without secreting the target substance: performing an action of producing a target substance and recovering the target substance from within the cells;
· a property of secreting a target substance extracellularly: performing an action of producing a target substance and recovering the target substance from the culture medium;
· a property of enabling easy culture accompanied by production of a target substance: performing an action of producing a target substance under general culture conditions;
· a property of being able to produce a plurality of target substances in a desired ratio: performing an action of producing a plurality of target substances;
· a property of producing a target substance without producing harmful substances: performing an action of producing a target substance without using special protective equipment;
· a property of having robust production of a target substance: performing an action of producing a target substance at a constant ratio;
· a property in which no deletion occurs in a gene for producing a target substance during a culture period: performing an action of producing a target substance with a strain in which no gene deletion occurs;
· a property of enabling control of production of a target substance: performing an action of producing a target substance under desired conditions (production and type or ratio of derivatives);
· a property of being able to suppress production of substances other than a target substance: performing an action of producing only a target substance;
· by-products that are difficult to separate by affinity, ion exchange, gel filtration, reversed phase, normal phase, chiral chromatography, distillation, or crystallization;
· performing an action of producing only a substance having a desired structure (without producing derivatives);
· a property of producing a small amount of empty capsids in viral vector production: performing an action of producing viral capsids containing a desired nucleic acid; and
· a property in which no deletion occurs in a plasmid: performing an action of recovering a plasmid having a target sequence.

### (Item X5)

The method according to any one of the preceding items, further including a step 4) of subjecting the construct of a single strain or a plurality of strains selected in the step 3) to combinatorial library construction and introducing the construct into a target host cell; and 5) a step of performing the step 3) again.

### (Item X5-1)

The method according to any one of the preceding items, in which the combinatorial library construction includes constructing a combinatorial plasmid library in which promoter sequence portions in the plasmid are shuffled.

### (Item X6)

A method for producing a substance in a host organism, the method including:
a step (A) of constructing a construct in which a promoter introduction site and a sequence for reconstruction are added to a gene cluster involved in production of the substance having undesired restriction enzyme recognition sequences removed;
a step (B) of introducing, into a host organism for combinatorial library construction, a plurality of types of constructs in which promoters are introduced into the promoter introduction sites;
a step (C) of performing combinatorial library construction of promoter regions among the plurality of types of constructs by a Combi-OGAB^{™} method;
a step (D) of culturing a host organism for production having a construct forming a combinatorial library and selecting a plurality of strains based on any criterion;
a step (E) of performing combinatorial library construction again for the promoter regions in the constructs of the plurality of strains by a Combi-OGAB^{™} method; and
a step (F) of producing a substance by using a strain having a desired property.

### (Item X7)

The method according to any one of the preceding items, in which the host organism for combinatorial library construction and the host organism for production are the same.

### (Item X8)

The method according to any one of the preceding items, in which the host organism for combinatorial library construction and the host organism for production are different.

### (Item X9)

The method according to any one of the preceding items, in which the host organism is Bacillus subtilis.

### (Item X10)

A method for producing a substance in a host organism, the method including a step of producing the substance by using a strain produced by the method according to any one of the preceding items.

### (Item X11)

A method for producing a substance in a host organism, the method including a step of producing the substance by using a strain obtained through the steps (A) to (E) in the method according to any one of the preceding items.

### (Item X12)

The method according to any one of the preceding items, further including modifying the strain.

### (Item X13)

The method according to any one of the preceding items, further including a step of repeating the steps (D) and (E) multiple times.

### (Item X14)

The method according to any one of the preceding items, in which the construction of the construct in the step (A) is performed by an OGAB^{™} method.

### (Item X15)

The method according to any one of the preceding items, in which the promoters are a plurality of promoters different from one another in transcription initiation timing and/or transcription strength.

### (Item X16)

The method according to any one of the preceding items, in which the host organism for production is Bacillus subtilis, actinomycetes, filamentous fungi, yeast, hydrogen-oxidizing bacteria, coryneform bacteria, or animal cells.

### (Item X17)

The method according to any one of the preceding items, in which the host organism is Bacillus subtilis.

### (Item X18)

The method according to any one of the preceding items, in which the undesired restriction enzyme recognition sequences are Type IIS restriction enzyme recognition sequences selected from the group consisting of AarI, BsaI, BbsI, BsmBI, and BspQI, and an SfiI recognition sequence for reconstruction.

### (Item X19)

The method according to any one of the preceding items, in which the sequence for reconstruction is an SfiI recognition sequence.

### (Item X20)

The method according to any one of the preceding items, in which the gene cluster is a gene cluster of polyketide synthases (PKSs) and non-ribosomal peptide synthases (NRPSs).

In addition, the gist of the present disclosure is further as follows.

### (Item 1)

A method for producing a strain having a desired property, the method including:
a step 1) of providing a construct into which a plurality of promoters are introduced, the construct including a gene cluster containing a plurality of genes;
a step 2) of performing combinatorial library construction for the promoters in the construct; and
a step 3) of selecting, in a host cell, a strain having a desired property.

### (Item 2)

The method according to the preceding item, in which the plurality of promoters are arranged in a monocistronic configuration.

### (Item 3)

The method according to any one of the preceding items, in which the strain having the desired property has at least one characteristic selected from the group consisting of:
· a strain capable of producing a target substance;
· a strain that highly produces a target substance;
· a strain that retains a target substance within the cells without secreting the target substance;
· a strain that secretes a target substance extracellularly;
· a production strain for a target substance that is easy to culture;
· a strain capable of producing a plurality of target substances in a desired ratio;
· a strain that produces a target substance without producing harmful substances;
· a strain having robust production of a target substance;
· a strain in which no deletion occurs in a gene for producing a target substance during a culture period;
· a strain for which control of production of a target substance is possible;
· a strain capable of suppressing production of substances other than a target substance;
· by-products that are difficult to separate by affinity, ion exchange, gel filtration, reversed phase, normal phase, chiral chromatography, distillation, or crystallization;
· a strain capable of suppressing production of impurities;
· a strain having a low amount of empty capsid production in viral vector production; and
· a strain in which no deletion occurs in a plasmid.

### (Item 4)

A method including performing an action based on the desired property by using a strain produced according to any one of the preceding items.

### (Item 5)

The method according to any one of the preceding items, further including a step 4) of subjecting the construct of a single strain or a plurality of strains selected in the step 3) to combinatorial library construction and introducing the construct into a target host cell; and 5) a step of performing the step 3) again.

### (Item 6)

A method for producing a substance in a host organism, the method including:
a step (A) of constructing a construct in which a promoter introduction site and a sequence for reconstruction are added to a gene cluster involved in production of the substance having undesired restriction enzyme recognition sequences removed;
a step (B) of introducing, into a host organism for combinatorial library construction, a plurality of types of constructs in which promoters are introduced into the promoter introduction sites;
a step (C) of performing combinatorial library construction of promoter regions among the plurality of types of constructs by a Combi-OGAB^{™} method;
a step (D) of culturing a host organism for production having a construct forming a combinatorial library and selecting a plurality of strains based on any criterion;
a step (E) of performing combinatorial library construction again for the promoter regions in the constructs of the plurality of strains by a Combi-OGAB^{™} method; and
a step (F) of producing a substance by using a strain having a desired property.

### (Item 7)

The method according to any one of the preceding items, in which the host organism for combinatorial library construction and the host organism for production are the same.

### (Item 8)

The method according to any one of the preceding items, in which the host organism for combinatorial library construction and the host organism for production are different.

### (Item 9)

The method according to any one of the preceding items, in which the host cell is Bacillus subtilis.

### (Item 10)

A method for producing a substance in a host organism, the method including a step of producing the substance by using a strain produced by the method according to any one of the preceding items.

### (Item 11)

A method for producing a substance in a host organism, the method including a step of producing the substance by using a strain obtained through the steps (A) to (E) in the method according to any one of the preceding items.

### (Item 12)

The method according to any one of the preceding items, further including modifying the strain.

### (Item 13)

The method according to any one of the preceding items, further including a step of repeating the steps (D) and (E) multiple times.

### (Item 14)

The method according to any one of the preceding items, in which the construction of the construct in the step (A) is performed by an OGAB^{™} method.

### (Item 15)

The method according to any one of the preceding items, in which the promoters are a plurality of promoters different from one another in transcription initiation timing and/or transcription strength.

### (Item 16)

The method according to any one of the preceding items, in which the host organism for production is Bacillus subtilis, actinomycetes, filamentous fungi, yeast, hydrogen-oxidizing bacteria, coryneform bacteria, or animal cells.

### (Item 17)

The method according to any one of the preceding items, in which the host organism is Bacillus subtilis.

### (Item 18)

The method according to any one of the preceding items, in which the undesired restriction enzyme recognition sequences are Type IIS restriction enzyme recognition sequences selected from the group consisting of AarI, BsaI, BbsI, BsmBI, and BspQI, and an SfiI recognition sequence for reconstruction.

### (Item 19)

The method according to any one of the preceding items, in which the sequence for reconstruction is an SfiI recognition sequence.

### (Item 20)

The method according to any one of the preceding items, in which the gene cluster is a gene cluster of polyketide synthases (PKSs) and non-ribosomal peptide synthases (NRPSs).

According to one embodiment of the present disclosure, in order to transcriptionally activate a desired gene cluster such as a natural product biosynthetic enzyme gene cluster in monocistronic units, to perform combinatorial library construction of promoters for the respective genes, and to run screening cycles, various restriction enzymes can be used. In one embodiment of the present disclosure, for long and complex sequences such as natural product biosynthetic enzyme gene clusters, cloning from the genome of the target microorganism had conventionally been the only available method, and removing restriction enzyme recognition sequences one by one had been an extremely time-consuming and costly operation; however, it is possible to perform such work in a simplified manner and to run screening cycles. In one embodiment of the present disclosure, even in cases where a new gene pathway is added to a natural biosynthetic system or gene clusters related to biopharmaceutical synthesis or sequence optimization of AAV vectors often include long and complex sequences, it is possible to run screening cycles easily and apply the present disclosure in such situations.

In one embodiment of the present disclosure, the OGAB^{™} method can be used, and this method is an innovative technique that assembles numerous DNA fragments and synthesizes long-chain DNA. In one application of one embodiment of the present disclosure, this technology can be used to construct, on a computer, long-chain gene clusters from which restriction enzyme recognition sequences such as those described above are removed. In one application of one embodiment of the present disclosure, by using the OGAB^{™} method, it is possible to incorporate a promoter introduction site into the design so that promoters are not contained at the time of construction and can instead be introduced later. By employing this strategy, it is not necessary to assemble numerous DNA fragments for each plasmid (seed plasmid) into which a promoter serving as a seed for combinatorial libraries is introduced, and a promoter can be introduced into a plasmid constructed by the OGAB^{™} method. Furthermore, even in cases where it is desired to add a specific promoter type depending on the experimental results, a seed plasmid can be easily prepared, and this advantage can also be applied in one embodiment of the present disclosure.

In the production of a target substance, the target substance, intermediates in the metabolic pathway, or proteins produced from constituent genes may affect the growth of the host organism (for example, antibiotics or cytotoxicity). In such a case, it is expected that self-resistance is acquired by adjusting so that production occurs in the late growth phase, and production of the substance occurs (Reference 5). Although it is not possible to predict, for each gene constituting a gene cluster, at which growth phase and with what transcription strength transcription should occur in order to be appropriate for producing a target substance, in one embodiment of the present disclosure, a combinatorial library of growth-phase-dependent promoters having different strengths is constructed for each gene in addition to a promoter that constantly exhibits transcriptional activity, and by performing screening using the production level as an index, a strain having the optimal combination of promoters can be obtained.

There is a Bacillus subtilis strain used in one embodiment of the present disclosure, and this microorganism naturally produces NRPS products such as surfactin and plipastatin, and has high potential as a host for producing natural products. In one embodiment of the present disclosure, the OGAB^{™} method can be performed using Bacillus subtilis, and by using this method, when a growth-phase-dependent promoter of Bacillus subtilis (Reference 6) is introduced, the Bacillus subtilis used for assembling the gene cluster into which the promoter is introduced can be used directly for substance production. As a result, steps such as plasmid extraction and transformation into a host can be omitted, enabling rapid acquisition of a strain that produces the target substance.

### Advantageous Effects of Invention

According to the present disclosure, it is possible to obtain a strain exhibiting a desired property rapidly and efficiently. The present disclosure can also provide a method for producing a microbial strain exhibiting a desired property such as high production and suppression of by-products by activating a desired gene cluster, such as genes constituting a natural product biosynthetic enzyme gene cluster, and through the method, can provide a substance that may be novel.

### Brief Description of Drawings

[FIG. 1] FIG. 1 is a diagram illustrating the structure of the assembly plasmid vector pGETS151 deltaBsmBI-pBR.
[FIG. 2] FIG. 2 is a diagram illustrating the plasmid in which Triketide Pyrone BGC used in Example 1 is assembled.
[FIG. 3] FIG. 3 is a diagram illustrating a state in which the production of Pyrone-OMe converges according to the Combi-OGAB^{™} cycle.
[FIG. 4] FIG. 4 is a diagram illustrating a state in which the methylation efficiency by BpsB increases and converges according to the Combi-OGAB^{™} cycle.
[FIG. 5] FIG. 5 is a diagram illustrating the plasmid in which Gramicidin S BGC used in Example 2 is assembled.
[FIG. 6] FIG. 6 is a diagram illustrating a chromatogram of strains in the 1st cycle of Combi-OGAB^{™} that produce Gramicidin S as a by-product.
[FIG. 7] FIG. 7 is a diagram illustrating a state in which the production of Gramicidin S converges according to the Combi-OGAB^{™} cycle.
[FIG. 8] FIG. 8 is a spectrum obtained by LC-MS analysis of the product of a strain that produces Gramicidin S as a by-product.

### Description of Embodiments

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

In the present specification, a promoter being "arranged in a monocistronic configuration" refers to the arrangement in which a single promoter is placed for a single gene. On the other hand, the phrase "arranged in a polycistronic configuration" refers to the arrangement in which a single promoter is placed for a plurality of genes.

In the present specification, the term "combinatorial library construction" refers to creating a library by preparing exhaustive combinations of a plurality of types of substances (for example, a plurality of types of promoters). For example, the combinatorial library construction includes constructing a combinatorial plasmid library in which promoter sequence portions in the plasmid are shuffled.

In the present specification, the term "desired property" refers to a beneficial property in substance production. Examples of the desired property include a property of being able to produce a target substance; a property of highly producing a target substance; a property of retaining a target substance within the cells without secreting the target substance; a property of secreting a target substance extracellularly; a property of enabling easy culture accompanied by production of a target substance (for example, growing under general culture conditions (such as media, temperature, and culture time)); a property of being able to produce a plurality of target substances in a desired ratio; a property of producing a target substance without producing harmful substances (for example, having no toxicity); a property of having robust production of a target substance; a property in which no deletion occurs in a gene for producing a target substance during a culture period; a property of enabling control of production of a target substance; a property of being able to suppress production of substances other than a target substance; a property of being able to suppress production of by-products and/or impurities that are difficult to separate by affinity, ion exchange, gel filtration, reversed phase, normal phase, chiral chromatography, distillation, or crystallization; a property of producing a small amount of empty capsids in viral vector production (for example, an empty capsid ratio of 80% or less, preferably 70% or less, more preferably 60% or less, 50% or less, 40% or less, 30% or less, 20% or less, or 10% or less, and most preferably 0%); and a property in which no deletion occurs in a plasmid.

In the present specification, the term "property of highly producing a target substance" refers to a property in which the production level is higher than that of a natural strain. The natural strain refers to a strain in which a substance-producing gene cluster that has not been sequence-engineered (has not been subjected to promoter replacement or the like) is introduced into a production host (as described in Claim 16). The property of highly producing a target substance can be a property in which the production level is at least 10% or higher, and preferably at least 20% or higher, than that of the natural strain, but is not limited thereto.

In the present specification, the term "property of enabling easy culture accompanied by production of a target substance" refers to a property in which a strain grows and produces a target substance under culture conditions generally used for the strain. For example, this indicates that the strain is "not difficult to culture", in that it can be cultured under general culture conditions, such as shaking culture for 24 hours at 37°C using a medium commonly used for microbial culture, such as LB medium, which is a non-selective medium. In addition, conditions in which the strain can be cultured in a biosafety level (BSL) 1 laboratory, which is a general laboratory, rather than in BSL2 or BSL3 facilities that require special equipment from the standpoint of pathogenicity or the like for handling the strain, are also encompassed in the ease of culture.

In the present specification, the term "property of having robust production of a target substance" refers to a highly reproducible property in which an equivalent production level is consistently exhibited when production is examined. As one method for verifying robustness, the robustness of experimental results can be evaluated by a calculation using the Fano factor described, for example, in ACS Synth. Biol., 11, 1686-1691 (2022).

In the present specification, the term "property in which no deletion occurs in a gene" refers to a property in which neither deletion of all or part of the nucleic acid encoding the gene, resulting in deletion of the gene or a portion thereof, nor loss of gene function caused by a mutation in part of the nucleic acid encoding the gene occurs.

In the present specification, the phrase "removing an undesired restriction enzyme recognition sequence" refers to deleting the undesired restriction enzyme recognition sequence in the method of the present disclosure, substituting the undesired restriction enzyme recognition sequence with another base or sequence, adding another base or sequence so as to eliminate the undesired restriction enzyme recognition sequence, or altering the undesired restriction enzyme recognition sequence into a sequence that cannot be recognized by the restriction enzyme. The undesired restriction enzyme recognition sequence may be a recognition sequence for a restriction enzyme used in DNA synthesis by the OGAB^{™} method or in library construction by the Combi-OGAB^{™} method.

In the present specification, the term "promoter introduction site" refers to a site upstream of each gene in a gene cluster at which a promoter is to be introduced.

In the present specification, the term "sequence for reconstruction" refers to a restriction enzyme recognition sequence used for forming an assembled nucleic acid. The assembled nucleic acid formed by cleavage is reconstructed to generate a circular plasmid.

In the present specification, the term "OGAB^{™} (Ordered Gene Assembly in Bacillus subtilis) method" or "method for assembling multiple DNA fragments using Bacillus subtilis" refers to a method for producing a circular plasmid in a transgenic organism by allowing the organism to take up an assembled nucleic acid. The OGAB^{™} method can use any organism capable of taking up a non-circular long-chain nucleic acid and producing a plasmid, and such an organism is referred to as a "transgenic organism" in the present specification. In the OGAB^{™} method, a plasmid containing a large sized nucleic acid can be conveniently prepared. The nucleic acid to be incorporated into the transgenic organism is referred to as "assembled nucleic acid" in the present specification, typically, the assembled nucleic acid has a tandem repeat-like structure in which one unit of the plasmid and one set of unit nucleic acids repeatedly appear in the same direction, and the use of the assembled nucleic acid having such a structure can promote the production of the plasmid in the transgenic organism.

In the present specification, the term "Combi-OGAB^{™} method", "combinatorial OGAB^{™} method", and "method for constructing a combinatorial library using Bacillus subtilis" refer to a method in which a mixture of assembled nucleic acids derived from a plurality of constructs is reconstructed into combinations of unit nucleic acids by the OGAB^{™} method to create a combinatorial library. Reference is also made to JP 7101431 B2. Note that "OGABR^{™}" and "Combi-OGAB^{™}" are trademarks of the present applicant.

In the present specification, the term "actinomycetes" refers to gram-positive bacteria that exhibit morphological characteristics in which cells form hyphae and grow in an elongated manner, and broadly includes many genera under the phylum Actinobacteria (Actinomycetota), and based on molecular phylogeny using the base sequence of the 16S ribosomal RNA gene (16S rRNA gene), certain rod-shaped bacteria and cocci are also classified as actinomycetes.

In the present specification, the term "filamentous fungi" refers to fungi that form hyphae and proliferate by spores, which are generally referred to as molds.

In the present specification, the term "polyketide synthase (PKS)" refers to a multidomain enzyme or enzyme complex that synthesizes polyketides. Polyketide synthases are possessed by eubacteria, fungi, plants, and a small number of animals. There are three types of PKSs, including Type I PKSs, which are large proteins in which a plurality of domains are arranged on a single polypeptide; Type II PKSs, which are complexes of proteins with different functions; and Type III polyketide synthases, which are small proteins composed only of a ketosynthase domain. All of these enzymes catalyze reactions in which an extender substrate (such as malonyl-CoA) is repeatedly condensed with a starter substrate, which is a CoA ester (or ACP form). As the starter substrate, acetyl-CoA, a fatty acid CoA ester, benzoyl-CoA, coumaroyl-CoA, and the like are usually used.

In the present specification, the term "non-ribosomal peptide synthase (NRPS)" refers to a group of enzymes that synthesize peptides using amino acids as substrates without depending on ribosomes and without using nucleic acids such as mRNA. The entity of NRPS is a complex of ultra-large proteins whose molecular weights reach several thousand kDa.

In the present specification, the term "Bacillus subtilis" refers to any non-pathogenic bacterium with a natural transformation ability, having the scientific name of Bacillus subtilis or including the genus Bacillus, that is closely related thereto, such as Bacillus amyloliquefaciens, Bacillus licheniformis, or Bacillus pumilus, which is considered to be an aerobic, gram-positive, and catalase-positive bacterium that is generally present in soil and plants and is present in the gastrointestinal tract of ruminants and humans, has a size of 0.7 to 0.8 × 2 to 3 µm, is mesophilic, has an optimal growth temperature of 25 to 40°C, and forms spores. In a preferred embodiment, Bacillus subtilis is Marburg 168 strain or its derivative strain RM125, which is a strain with high natural transforming potential among Bacillus subtilis. Because strain 168 is the most genetically studied gram-positive bacterium, is itself harmless to humans and animals, and is known to be applicable to the OGAB^{™} method, the strain 168 can be suitably used in the present disclosure.

In the present specification, the term "sequence replicated in Bacillus subtilis" is used in the same sense as "a sequence amplified in Bacillus subtilis" and refers to a nucleic acid sequence that is replicated (or amplified) when it is in a state of being introduced into Bacillus subtilis. Herein, in this context, the amplification is used in the same sense as "replication". Examples of a replication mechanism of nucleic acid (plasmid) in Bacillus subtilis include rolling circle type, theta type, oriC, and phage, and any mechanism can be used as a sequence to be replicated in Bacillus subtilis. The rolling circle type replication mechanism is a mechanism in which one strand of a double-stranded DNA is replicated and then the other strand is replicated, and the plasmid tends to be destabilized because the time during which the nucleic acid exists as a single-stranded DNA is long. The theta type replication mechanism is a mechanism in which replication of a double-stranded DNA (plasmid) is simultaneously initiated in two directions from an origin of replication as in the case of replication of a bacterial chromosome, and can be preferably used in replication of a long DNA (for example, 10 kb or more) as in the present disclosure (Janniere, L., A. Gruss, and S. D. Ehrlich. 1993. Plasmids, p. 625-64. InA. L. Sonenshein, J. A. Hoch, and R. Losick (ed.), Bacillus subtilis and other Gram-positive bacteria: biochemistry, physiology and molecular genetics. American Society for Microbiology, Washington, D.C.). The oriC replication mechanism operates in the same manner as in replication of a host bacterial (for example, Bacillus subtilis) chromosome. Examples of the sequences that are replicated in Bacillus subtilis include a plasmid or a portion thereof, or an origin of replication or a variant thereof, which is known to activate the replication mechanisms such as rolling circle type, theta type, oriC, and phage. As a rolling circle type plasmid, pUB110, pC194, pE194, pT181 and the like are known, and as a theta type plasmid, pAMβ1, pTB19, pLS32, pLS20, and the like are known. The sequences that are replicated in Bacillus subtilis may contain a sequence that is identical or similar to a known origin of replication (for example, 90% or more, 95% or more, 97% or more, 98% or more, 99% or more, or 99.5% or more sequence identity). For example, in a case where a DNA fragment in which a candidate DNA fragment and a selectable marker gene effective in Bacillus subtilis, such as a drug-resistant gene, are linked is introduced into Bacillus subtilis and then cultured (with addition of a drug or the like), and then extrachromosomal replication is performed, it can be determined that the candidate DNA fragment contains sequences that are replicated in Bacillus subtilis.

As used in the present specification, the term "plasmid" refers to a circular DNA that is present separately from chromosomes in a cell or that is present separately from chromosomes when introduced into a cell.

In the present specification, the term "nucleic acid sequence that promotes plasmid replication" is used in the same sense as "nucleic acid sequence that promotes plasmid amplification", and refers to any nucleic acid sequence that, when introduced into a host cell (for example, Bacillus subtilis), promotes replication (in this context, it is the same as amplification) of a plasmid present in the host cell. Preferably, the nucleic acid sequence that promotes plasmid replication is operably linked to, but not limited to, a nucleic acid sequence encoding a target plasmid. Details of the nucleic acid sequence that promotes plasmid replication, the target plasmid, the relationship therebetween, and the like, are described elsewhere in the present specification. Examples of the nucleic acid sequence that promotes plasmid replication include a nucleic acid sequence containing an origin of replication that operates in a target host cell (for example, Bacillus subtilis). A nucleic acid sequence that promotes plasmid amplification is a DNA fragment containing an origin of replication, and refers to a DNA fragment that can replicate independently of chromosomal DNA. Whether or not these DNA fragments can be replicated can be confirmed by a method in which a selectable marker gene such as a drug-resistant gene is linked to these DNA fragments, the resulting product is cultured under selective conditions such as a drug, plasmid DNA is then purified, and a band of the DNA is observed by electrophoresis. In addition to the origin of replication, the plasmid may contain a gene for a rep protein that induces the host's DNA replication enzymes to the origin of replication, a segregation mechanism gene for ensuring segregation of the plasmid into daughter cells, a selectable marker gene, and the like. The origin of replication may be fused within the gene for the rep protein.

As used in the present specification, the term "packaging cell" refers to a cell for producing a vector plasmid.

As used in the present specification, the term "vector plasmid" or "viral vector plasmid" refers to a plasmid for producing a viral vector in a producer cell, the plasmid containing a gene to be carried on the viral vector. In one embodiment, the vector plasmid may contain a sequence of a gene (desired gene) to be expressed in a target cell of the viral vector between two terminal repeat sequences, may additionally contain a promoter, and may further contain other elements (for example, an enhancer, a terminator, and the like).

As used in the present specification, the term "producer cell" refers to a cell capable of producing a desired viral vector, and may be a cell in which genes necessary for production of the viral vector are expressed from the chromosome and an introduced plasmid. A desired viral vector can be produced by introducing the vector plasmid of the present disclosure into the producer cell. For example, in the case of an AAV viral vector, E1A and E1B are required for its production; however, because HEK293 cells possess these factors, E1A and E1B can be omitted from the helper plasmid. Other cells can also function as producer cells when they are modified to have such helper factors.

As used in the present specification, the term "viral vector" refers to a construct that has at least a portion of a structure derived from a virus and is capable of introducing a nucleic acid into a target cell. Typically, a viral vector is in the form of a viral particle containing a capsid of the virus and a nucleic acid containing a heterologous gene. As used in the present specification, the term "origin virus" refers to a virus that naturally possesses the viral-derived structure included in the viral vector. In a viral vector, the nucleic acid encapsulated in the capsid (loaded nucleic acid) contains, between two terminal repeat sequences, a sequence of a gene (desired gene) to be expressed in a target cell of the viral vector, and may additionally contain a promoter, an enhancer, a terminator, and the like, and may also contain a gene derived from the origin virus.

In the present specification, the term "nucleic acid sequence required to constitute the viral vector" refers to a nucleic acid sequence (for example, a combination of nucleic acid sequences) that enables a producer cell containing the sequence to produce the viral vector. In one embodiment, the nucleic acid sequences required to constitute the viral vector include a nucleic acid sequence encoding a capsid protein of the virus, a nucleic acid sequence encoding a protein that packages, transcribes, and replicates the genome of the virus, two terminal repeat sequences of the virus, and a nucleic acid sequence of helper genes. The term "nucleic acid sequence of helper genes" may include, for example, a nucleic acid sequence encoding a capsid protein of a virus, a nucleic acid sequence encoding a protein that packages, transcribes, and replicates the genome of the virus, and any nucleic acid sequence required to constitute the viral vector other than the two terminal repeat sequences of the virus, or may essentially consist of such sequences. Each of these nucleic acid sequences may differ depending on the viral vector. Details thereof are described elsewhere in the present specification. In a preferred embodiment, the nucleic acid sequences required to constitute the viral vector employ advantageous sequences common to AAV and adenovirus, and more preferably employ sequences advantageous for AAV. Such advantageous sequences common to AAV and adenovirus contain a desired gene between inverted terminal repeat sequences (ITR) and may contain a helper gene, rep, cap, or L1, L2, L3, L4, and L5. Sequences advantageous for AAV contain a desired gene between ITR sequences and may contain a helper gene, rep, and cap. The arrangement of the helper gene, rep, and cap may be located outside the two ITRs. Examples of the helper genes include, but are not limited to, the sequences of specific helper genes such as E1A, E1B, E2A, E2B, E3, and E4.

In the present specification, the term "proteins that package, transcribe, and replicate the viral genome" refers to a protein that encapsulates the viral genome into a capsid, a protein that transcribes the viral genome, a protein that replicates the viral genome, and a protein having both functions, and examples thereof include E1A, E1B, E2A, E2B, E4, rep, pol, ψ, APP, MAAP, and rev. Without intending to be limiting, the proteins that package, transcribe, and replicate the viral genome may be derived from any of adenovirus serotypes 1 to 52 or adeno-associated virus serotypes 1 to 12, or from variants thereof (such as rh10, DJ, DJ/8, PHP.eB, PHP.S, AAV2-retro, AAV2-QuadYF, AAV2.7m8, AAV6.2, rh.74, AAV2.5, AAV-TT, and Anc80). These proteins may be natural or may have artificial mutations introduced therein.

As used in the present specification, the term "capsid" refers to a protein produced from a viral gene that is present on the surface of a virus or viral vector (and, as necessary, enclosed within an envelope), and is also referred to as a "capsid protein". The capsid may be responsible for infectivity to the cell. Examples of capsid proteins include, but are not limited to, L2, L3, cap, VSV-G, and gag. Without intending to be limiting, nucleic acid sequences encoding a viral capsid protein may be derived from any of adenovirus serotypes 1 to 52 or adeno-associated virus serotypes 1 to 12, or from variants thereof (such as rh10, DJ, DJ/8, PHP.eB, PHP.S, AAV2-retro, AAV2-QuadYF, AAV2.7m8, AAV6.2, rh.74, AAV2.5, AAV-TT, and Anc80). The capsid may be natural or may have artificial mutations introduced therein.

In the present specification, the "repeat sequence" or "tandem repeat" (a nucleic acid sequence) is a generic term for a nucleic acid sequence of an organism genome in which the same sequence is repeatedly (particularly several times or more) found. Any repeat sequence used in the art can be used in the present disclosure. Typically, a promoter sequence appears repeatedly.

In the present specification, the term "terminal repeat sequence" refers to a nucleic acid sequence in an organism's genome in which the same sequence is repeatedly (particularly several times or more) observed and that is present at a terminal region. Examples of the terminal repeat sequence include, but are not limited to, an inverted terminal repeat sequence (ITR) or a long chain terminal repeat sequence (LTR). The terminal repeat sequence may be derived from any of adenovirus serotypes 1 to 52 or adeno-associated virus serotypes 1 to 12, or from variants thereof (such as rh10, DJ, DJ/8, PHP.eB, PHP.S, AAV2-retro, AAV2-QuadYF, AAV2.7m8, AAV6.2, rh.74, AAV2.5, AAV-TT, and Anc80). The terminal repeat sequence may be natural or may have artificial mutations introduced therein.

In the present specification, the term "helper gene" refers to a gene that assists the amplification of a virus that cannot replicate on its own. In the present disclosure, examples of the helper genes include, but are not limited to, a nucleic acid sequence encoding a capsid protein of the virus; a nucleic acid sequence encoding a protein that packages, transcribes, and/or replicates the genome of the virus; and any nucleic acid sequence other than the two terminal repeat sequences of the virus that is required to constitute or propagate the viral vector, to enhance its activity, or to reduce its toxicity. Examples of the helper genes that may be used include, but are not limited to, E1A, E1B, E2A, E2B, E4, RPE, WRPE, PPT, oPRE, enhancer sequences, insulator sequences, and silencer sequences.

In the present specification, the term "unit nucleic acid" refers to a nucleic acid molecule or a portion of a nucleic acid molecule having a part of a sequence constituting the sequence of the assembled nucleic acid, and as described in detail elsewhere in the present specification, a plurality of types of unit nucleic acids are prepared as a unit vector, and then the assembled nucleic acid is constructed. The unit nucleic acid of the present disclosure may be DNA, RNA, a variant thereof, or a mixture thereof. A method for preparing the unit nucleic acid of the present disclosure or a composition containing the unit nucleic acid includes a step of preparing solutions each containing a plurality of unit nucleic acids to which an additional sequence is ligated; and, after preparation of the respective solutions, a step of measuring the concentration of the unit nucleic acids in each solution in a state in which the additional sequence is ligated to the unit nucleic acids, and, based on the measurement results, aliquoting each solution so as to make the molar amounts of the unit nucleic acids in the respective solutions approach one another. In the present specification, the types of "unit nucleic acids" are distinguished according to their respective base sequences. In addition, the "unit nucleic acid" includes both those to which a restriction enzyme recognition site is added and those to which a restriction enzyme recognition site is not added. The length of each unit nucleic acid is not particularly limited; however, it is preferable that fewer base sequencing operations are required when confirming the base sequence of the unit nucleic acid, since this can reduce temporal and monetary cost. Therefore, it is preferable that the length of each unit nucleic acid is shorter; specifically, it is preferable that the length is 1,600 bp or less, and more preferably 1,200 bp or less. In particular, when base sequencing is performed using an automated fluorescent sequencer employing the Sanger method, it is possible to confirm a continuous base sequence of about 800 bases in a single sequencing run; therefore, it is most preferable that the length of each unit nucleic acid is 800 bp or less (specifically, 600 bp or less, 500 bp or less, 400 bp or less, 200 bp or less, 100 bp or less, and the like). As described above, when the length of each unit nucleic acid is short, a large number of unit nucleic acids is required when these are used for preparation of a DNA concatemer described below. However, when the unit nucleic acids prepared by the method of the present disclosure are used, a large number of unit nucleic acids can be ligated, as described below. In addition, when the length of each unit nucleic acid is too short, the number of unit nucleic acids increases, and the work efficiency decreases. Therefore, the length of each unit nucleic acid is preferably 20 bp or more, more preferably 30 bp or more, and still more preferably 50 bp or more. The vector DNA and each unit nucleic acid have a structure capable of being repeatedly ligated to one another while maintaining their order. In the present specification, the phrase "ligating while maintaining their order with respect to one another" refers to unit nucleic acids or vector DNA that have adjacent sequences on an assembled nucleic acid unit being ligated while maintaining their order and orientation.

In the present specification, the term "endotoxin" refers to a toxin that is also called an internal toxin, which is a component of the cell wall of gram-positive bacteria such as Bacillus subtilis and is not actively secreted. Quantification can be performed using kits such as the Pierce LAL Endotoxin Quantitation Kit (Thermo Fisher Scientific, USA). "Endotoxin" is present only in gram-negative bacteria and is not present in gram-positive bacteria. "Endotoxin" also called lipopolysaccharide (LPS), is present in the outer membrane of gram-negative bacteria and has a structure consisting of an O-antigen, a core polysaccharide, and lipid A, among which lipid A is the physiologically active component. Gram-positive bacteria do not have an outer membrane; therefore, lipid A is also absent.

As used in the present specification, the term "loaded nucleic acid" refers to a nucleic acid carried by a viral vector. Whether a nucleic acid is a loaded nucleic acid can be confirmed by treating a viral vector preparation with DNase to degrade nucleic acids outside the capsid, inactivating the DNase, and subsequently examining the nucleic acid extracted from within the capsid. Whether a nucleic acid is a loaded nucleic acid can also be confirmed by examining the sequence of the resulting nucleic acid product (preferably the full-length sequence or a sequence close to full length).

As used in the present specification, the term "host cell" refers to a cell into which an exogenous nucleic acid, protein, virus, or viral vector is introduced (including the progeny of such a cell).

In the present specification, the terms "protein", "polypeptide", and "peptide" are used in the same meaning, and refer to an amino acid polymer of any length. The polymer may be linear, branched, or cyclic. An amino acid may be a natural, non-natural, or modified amino acid. The term also encompasses a natural or artificially modified polymer. Examples of such a modification include disulfide bond formation, glycosylation, lipidation, acetylation, phosphorylation, and any other manipulation or modification (for example, conjugation with a labeling component).

Unless otherwise indicated, a particular nucleic acid sequence is also intended to encompass a conservatively modified variant (for example, a degenerate codon substitution) and complementary sequence thereof, as well as the sequence explicitly indicated. Specifically, the degenerate codon substitution can be achieved by generating a sequence in which the third position of one or more selected (or all) codons is substituted with a mixed base and/or deoxyinosine residue. For example, in addition to known variants (such as rh10, DJ, DJ/8, PHP.eB, PHP.S, AAV2-retro, AAV2-QuadYF, AAV2.7m8, AAV6.2, rh.74, AAV2.5, AAV-TT, and Anc80), variants based on specific wild-type sequences, such as adenovirus serotypes 1 to 52 and adeno-associated virus serotypes 1 to 12, also include nucleic acids containing sequences having at least 70%, at least 80%, at least 85%, at least 90%, at least 95%, at least 96%, at least 97%, at least 98%, at least 99%, or at least 99.5% sequence identity to the original sequence.

In the present specification, the term "gene" refers to a nucleic acid moiety that performs a certain biological function. The biological function includes coding for polypeptides or proteins, coding for protein non-coding functional RNAs (rRNA, tRNA, microRNA (miRNA), lncRNA, and the like), regulating the production of polypeptides, proteins, or non-coding functional RNAs, specifically binding to specific proteins, and regulating cleavage or replication of nucleic acids. Therefore, in the present specification, the gene includes, in addition to a nucleic acid portion encoding a protein or a protein non-coding functional RNA, transcription/translation regulatory sequences such as a promoter, a terminator, an enhancer, an insulator, a silencer, an origin of replication, and an internal ribosome entry site, and nucleic acid portions required for packaging into a viral particle. In the present specification, the term "gene product" may refer to a polypeptide, a protein, or a protein non-coding functional RNA encoded by a gene.

In the present specification, when referring to the number of genes, one gene refers to a gene having a continuous sequence in the form in which it is normally (with the highest frequency or with a probability of 50% or more) present on the genome of an organism. For example, two exons encoding one protein may be two genes. For example, when a promoter sequence and a sequence encoding a protein form a continuous sequence, the nucleic acid portion including the promoter sequence and the sequence encoding the protein may be one gene. For example, when a protein that becomes functional by being cleaved is encoded on the genome by a continuous sequence, the protein may be encoded by one gene. When referring to a gene in terms of function, the nucleic acid sequence does not need to be a continuous sequence, and, for example, a plurality of exons encoding one protein are collectively referred to as the gene of the protein.

As used in the present specification, the term "deficient" of a gene refers to that a nucleic acid does not contain the gene or contains a gene that is modified so as not to perform the normal function (for example, the function of producing a functional protein) of the gene.

As used in the present specification, the phrase "operably linked" refers to placing the expression (actuation) of a desired sequence under the control of a transcriptional and translational regulatory sequence (for example, a promoter, an enhancer, or the like) or a translational regulatory sequence. In order for a promoter to be operably linked to a gene, the promoter is usually located immediately upstream of the gene, but is not necessarily located adjacent to the gene.

As used in the present specification, the term "transcription/translation regulatory sequence" collectively refers to promoter sequences, polyadenylation signals, transcription termination sequences, upstream regulatory domains, origins of replication, enhancers, IRESs, and the like, and these cooperate to enable replication, transcription, and translation of a coding sequence in a recipient cell. As long as replication, transcription, and translation of the selected coding sequence are possible in a suitable host cell, all of these transcription/translation regulatory sequences do not necessarily need to be present. Those skilled in the art can readily identify regulatory nucleic acid sequences from publicly available information. Furthermore, those skilled in the art can identify transcription/translation regulatory sequences applicable for the intended use, for example, in vivo, ex vivo, or in vitro.

As used in the present specification, the term "promoter" refers to a segment of a nucleic acid sequence that controls transcription of an operably linked nucleic acid sequence. A promoter contains a specific sequence that is sufficient for recognition, binding, and initiation of transcription by RNA polymerase. A promoter may also contain sequences that regulate recognition, binding, or initiation of transcription by RNA polymerase.

As used in the present specification, the term "enhancer" refers to a segment of a nucleic acid sequence that has a function of increasing the expression efficiency of a target gene.

As used in the present specification, the term "silencer" refers to a segment of a nucleic acid sequence that has a function of decreasing the expression efficiency of a target gene, in contrast to an enhancer.

As used in the present specification, the term "insulator" refers to a segment of a nucleic acid sequence that has a cis-regulatory function of regulating the expression of a gene located at a distant position on the DNA sequence.

As used in the present specification, the term "terminator" refers to a segment of a nucleic acid sequence that is located downstream of a region encoding a protein and is involved in termination of transcription when the nucleic acid is transcribed into mRNA.

As used in the present specification, the term "origin of replication" refers to a segment of a nucleic acid sequence to which a protein (for example, initiator DnaA protein or the like) that recognizes the nucleic acid sequence binds or to which RNA is synthesized to partially unwind a DNA double helix, and from which replication is initiated.

As used in the present specification, the internal ribosome entry site ("IRES") refers to a nucleic acid segment that promotes the entry or retention of a ribosome during translation of a downstream nucleic acid sequence.

In the present specification, the term "homology" of nucleic acids refers to a degree of identity of two or more nucleic acid sequences with respect to one another, and having "homology" generally refers to having a high degree of identity or similarity. Therefore, the identity or similarity of the sequences is higher as homology of two nucleic acids is high. The "similarity" is a numerical value calculated for a similar base in addition to identity, and here, the similar bases refer to a case where some bases in a mixed base (for example, R = A + G, M = A + C, W = A + T, S = C + G, Y = C + T, K = G + T, H = A + T + C, B = G + T + C, D = G + A + T, V = A + C + G, and N = A + C + G + T) are identical. Whether two types of nucleic acids have homology can be determined by a direct comparison of sequences or a hybridization method under stringent conditions. When two nucleic acid sequences are directly compared, the genes have homology when the genes are typically at least 50% identical, preferably at least 70% identical, and more preferably, at least 80%, 90%, 95%, 96%, 97%, 98%, or 99% identical between the nucleic acid sequences.

Amino acids may be mentioned in the present specification by either their commonly known three-letter symbols or their one-letter symbols recommended by the IUPAC-IUB Biochemical Nomen cloture Commission. Similarly, nucleotides may be mentioned by their commonly recognized one character codes. In the present specification, a comparison of similarity, identity, and homology of an amino acid sequence and a base sequence is calculated by using a sequence analysis tool BLAST and default parameters. For example, identity can be searched using BLAST 2.10.1+ (published on June 18, 2020) of the NCBI. In the present specification, a value for identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity.

In the present specification, unless otherwise specified, it is understood that reference to a biological material (for example, a protein, a nucleic acid, or a gene) is also a reference to a variant of the biological material (for example, a variant having a modification in an amino acid sequence) that performs a function similar to (but not to the same extent as) the biological function of the biological material. Such a variant can contain a fragment of the original molecule, and a molecule that is at least 30%, 40%, 50%, 60%, 70%, 80%, 90%, 95%, 98%, or 99% identical as compared to the sequence of the original molecule that is aligned over the amino acid sequence or nucleic acid sequence of the original biological material of the same size, or aligned by computer homology programs known in the art. A variant can contain a molecule with a modified amino acid (modification by, for example, disulfide bond formation, glycosylation, lipidation, acetylation, or phosphorylation) or a modified nucleotide (for example, modification by methylation).

In the present specification, the term "stringent conditions" refers to well-known conditions commonly used in the art. As the stringent conditions, for example, the following conditions can be adopted. (1) A low ionic strength and a high temperature for washing (for example, at 50°C, 0.015 M sodium chloride/0.0015 M sodium citrate/0.1% sodium dodecyl sulfate) are used, (2) a denaturant such as formamide during hybridization (for example, at 42°C, 50% (v/v) formamide and 0.1% bovine serum albumin/0.1% ficol/0.1% polyvinylpyrrolidone/50 mM sodium phosphate buffer with pH 6.5, 750 mM sodium chloride, 75 mM sodium citrate) is used, or (3) incubation is performed at 37°C overnight in a solution containing 20% formamide, 5 x SSC, 50 mM sodium phosphate (pH 7.6), 5 x Denhardt's solution, 10% dextran sulfate, and 20 mg/ml denatured sheared salmon sperm DNA, and then the filter is washed at about 37 to 50°C and 1 x SSC. Note that a formamide concentration may be 50% or more. A washing time may be 5, 15, 30, 60, or 120 minutes or longer. A plurality of factors such as a temperature and a salt concentration are considered as factors affecting the stringency of the hybridization reaction, and for details, reference can be made to Ausubel et al., Current Protocols in Molecular Biology, Wiley Interscience Publishers, (1995). Examples of the "highly stringent conditions" are 0.0015 M sodium chloride, 0.0015 M sodium citrate, and 65 to 68°C, or 0.015 M sodium chloride, 0.0015 M sodium citrate, 50% formamide, and 42°C. The operation can be performed by hybridization and methods described in experimental textbooks such as Molecular Cloning 2nd ed., Current Protocols in Molecular Biology, Supplement 1-38, DNA Cloning 1: Core Techniques, A Practical Approach, Second Edition, Oxford University Press (1995). Here, a sequence including only the A sequence or only the T sequence is preferably excluded from a sequence that hybridizes under stringent conditions. Moderately stringent conditions can be readily determined by those skilled in the art, for example, based on DNA length, are shown in Sambrook et al., Molecular Cloning: A Laboratory Manual, No. 3, Vol. 1, 7.42-7.45 Cold Spring Harbor Laboratory Press, 2001; and for nitrocellulose filters, include use of a pre-wash solution of 5 x SSC, 0.5% SDS, 1.0 mM EDTA (pH 8.0), hybridization conditions of about 50% formamide, 2 x SSC-6x SSC (or other similar hybridization solutions such as Stark's solution in about 50% formamide at about 42°C) at about 40 to 50°C, and wash conditions of 60°C, 0.5 x SSC, and 0.1% SDS. Thus, the polypeptides used in the present disclosure also include a polypeptide encoded by a nucleic acid molecule that hybridizes under highly or moderately stringent conditions to a nucleic acid molecule encoding a polypeptide specifically described in the present disclosure.

In the present specification, the term "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar moiety in an ortholog corresponding to a specific moiety of the antisense molecule. A corresponding amino acid can be a specific amino acid subjected to, for example, cysteinylation, glutathionylation, S-S bond formation, oxidation (for example, oxidation of a methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Accordingly, it is referred to in the present specification as a "corresponding" region or domain in such a case.

In the present specification, the term "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene may be an ortholog of such a gene. For example, the cap of AAV serotype 1 can correspond to the cap of AAV serotype 2. For example, a corresponding gene in a certain virus can be found by searching a sequence database of the virus using the gene sequence of a reference virus for the corresponding gene as a query sequence.

In accordance with the present disclosure, in the present specification, the term "activity" refers to a function of a molecule in its broadest sense. Activity, although not intended to be limiting, generally includes a biological function, biochemical function, physical function, and chemical function of a molecule. Examples of the activity include enzyme activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

In the present specification, when referring to a certain gene or a nucleic acid molecule or a polypeptide related thereto, the term "biological function" refers to a specific function that the gene, nucleic acid molecule, or polypeptide can have in vivo, and examples thereof include, but are not limited to, a specific cell surface structure recognition ability, an enzyme activity, and a binding ability to a specific protein. In the present disclosure, examples thereof include, but are not limited to, a function in which a certain promoter is recognized in a specific host cell. In the present specification, the biological function can be exerted by "biological activity". In the present specification, the term "biological activity" refers to an activity that a certain agent (for example, a polynucleotide, a protein, or the like) can have in a living body. Biological activity encompasses an activity of exerting a variety of functions (for example, transcription promoting activity), and also encompasses, for example, an activity of activating or inactivating another molecule by an interaction with a certain molecule. For example, when a certain factor is an enzyme, the biological activity thereof encompasses enzyme activity thereof. In another example, in a case where a certain factor is a ligand, binding to a receptor corresponding to the ligand is encompassed. Such biological activity can be measured by a technique that is well known in the art. Thus, the term "activity" refers to various measurable indicators that indicate or reveal binding (either directly or indirectly); and affect a response (that is, it has a measurable effect in response to some exposure or stimulus), and examples thereof include the amount of upstream or downstream proteins or a measure of other similar function in a host cell.

As used in the present disclosure, the term "infectivity" of a virus or viral vector refers to the ability of the virus or viral vector to introduce nucleic acid in the virus or viral vector into a cell by adhesion of the virus or viral vector to the cell or by membrane fusion. A Sendai viral vector may have the same replication ability as a wild-type vector, or may be attenuated by genetic mutation. The term "replication ability" of a virus or viral vector refers to the ability to produce infectious virus particles or viral vector particles in an infected cell.

As used in the present specification, the terms "transformation", "transduction", and "transfection" are used interchangeably, unless otherwise stated, and refer to the introduction of a nucleic acid into a host cell (optionally via a virus or a viral vector). As a transformation method, any method can be used as long as it is a method of introducing a nucleic acid into a host cell, and examples thereof include various well-known techniques such as use of competent cells, an electroporation method, a method using a particle gun (gene gun), and a calcium phosphate method.

In the present specification, the term "purified" substance or biological factor (for example, a nucleic acid, a protein, or the like) refers to a substance or a biological factor in which at least a part of a factor naturally accompanying the biological factor is removed. Thus, the purity of the biological factor in the purified biological factor is generally higher than the purity in the normal state of the biological factor (that is, concentrated). The term "purified" as used in the present specification refers to the presence of preferably at least 75% by weight, more preferably at least 85% by weight, still more preferably at least 95% by weight, and most preferably at least 98% by weight of a biological factor of the same type. The substance used in the present disclosure is preferably a "purified" substance.

In the present specification, a "drug", "agent", or "factor" (all the terms correspond to an "agent" in English) is used interchangeably in a broad sense, and may be any substance or other elements (for example, energy such as light, radioactivity, heat, and electricity) as long as the intended object can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that can be used as a medicine (for example, a small molecule ligand or the like), or the like), a composite molecule thereof, and a mixture thereof.

As used in the present specification, the "complex" or "complex molecule" means any construct containing two or more moieties. For example, when one moiety is a polypeptide, the other moiety may be a polypeptide or another substance (for example, a substrate, a sugar, a lipid, a nucleic acid, another hydrocarbon, or the like). In the present specification, two or more moieties constituting the complex may be bonded by a covalent bond or may be bonded by another bond (for example, a hydrogen bond, an ionic bond, a hydrophobic interaction, van der Waals force, or the like).

The term "about" refers to the indicated value plus or minus 10%. The term "about" when used for a temperature refers to an indicated temperature plus or minus 5°C, and the term "about" when used for a pH refers to an indicated pH plus or minus 0.5.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description in the present specification. In addition, it is also understood that the following embodiments may be used alone or in combination.

In one aspect, the present disclosure provides a method for producing a strain having a desired property, the method including: a step 1) of providing a construct into which a plurality of promoters are introduced, the construct including a gene cluster containing a plurality of genes; a step 2) of performing combinatorial library construction for the promoters in the construct; and a step 3) of selecting, in a host cell, a strain having a desired property. In this aspect, the plurality of promoters can be arranged in a monocistronic configuration.

In another aspect, the present disclosure provides a method for producing a substance in a host organism, the method including: a step (A) of constructing a construct in which a promoter introduction site and a sequence for reconstruction are added to a gene cluster involved in production of the substance having undesired restriction enzyme recognition sequences removed; a step (B) of introducing, into a host organism for combinatorial library construction, a plurality of types of constructs in which promoters are introduced into the promoter introduction sites; a step (C) of performing combinatorial library construction of promoter regions among the plurality of types of constructs by a Combi-OGAB^{™} method; a step (D) of culturing a host organism for production having a construct forming a combinatorial library and selecting a plurality of strains based on any criterion; a step (E) of performing combinatorial library construction again for the promoter regions in the constructs of the plurality of strains by a Combi-OGAB^{™} method; and a step (F) of producing a substance by using a strain having a desired property.

As examples of the arbitrary criteria, the criteria may be, for example, selecting strains having the highest production of the substance among the plurality of strains (for example, the top five), or selecting strains having a predetermined production level, and may be appropriately determined by those skilled in the art.

In one aspect, there is provided a method for producing a strain having a desired property, the method including: a step 1) of providing a plurality of constructs in which promoters are arranged in a monocistronic configuration, the construct including a gene cluster containing a plurality of genes; a step 2) of performing combinatorial library construction for the promoters in the construct and introducing the construct into a desired host cell; and a step 3) of selecting, in the host cell, a strain having a desired property.

In another aspect, the present disclosure provides a method for producing a substance in a host organism, the method including: a step (A) of constructing a construct in which a promoter introduction site and a sequence for reconstruction are added to a gene cluster involved in production of the substance having undesired restriction enzyme recognition sequences removed; a step (B) of introducing, into a host organism, a plurality of types of constructs in which promoters are introduced into the promoter introduction sites; a step (C) of performing, in cells of the host organism, combinatorial library construction of promoter regions among the plurality of types of constructs by a Combi-OGAB^{™} method; a step (D) of culturing the host organism and selecting a plurality of strains having a desired property; a step (E) of performing combinatorial library construction again for the promoter regions in the constructs of the plurality of strains having the desired property by a Combi-OGAB^{™} method; and a step (F) of producing a substance by using a strain having a desired property.

In still another aspect, the present disclosure provides a method for producing a substance in a host organism, the method including a step of producing the substance by using a strain produced by the method of the present disclosure.

In still another aspect, the present disclosure provides a method for producing a substance in a host organism, the method including a step of producing the substance by using a strain obtained through the steps (A) to (E) in the method of the present disclosure.

The method of the present disclosure may further include modifying the strain of the present disclosure. The modification may include, for example, introducing mutations or adding other genes such as a transporter and/or other metabolic enzymes. Such modification may be used to optimize the properties of the strain.

In one embodiment, examples of the strain having the desired property include, but are not limited to, a strain capable of producing a target substance, a strain that highly produces a target substance, a strain that retains a target substance within the cells without secreting the target substance, a strain that secretes a target substance extracellularly, a production strain for a target substance that is easy to culture (for example, growing under general culture conditions (such as media, temperature, and culture time)), a strain capable of producing a plurality of target substances in a desired ratio, a strain that produces a target substance without producing harmful substances (for example, having no toxicity), a strain having robust production of a target substance, a strain in which no deletion occurs in a gene for producing a target substance during a culture period, a strain that enables control of production of a target substance, a strain capable of suppressing production of substances other than a target substance, a strain capable of suppressing production of by-products and/or impurities that are difficult to separate by affinity, ion exchange, gel filtration, reversed phase, normal phase, chiral chromatography, distillation, or crystallization, a strain that produces a small amount of empty capsids in viral vector production, and a strain in which no deletion occurs in a plasmid, or any combination thereof.

In one embodiment, the strain having the desired property is at least a strain that highly produces a target substance, and may additionally have one or more of the above desired properties as needed.

### (Construction of the construct)

A nucleic acid containing a nucleic acid sequence for producing a viral vector and containing a sequence that replicates in cells can be introduced into a host cell, thereby allowing a plasmid to be formed in the host cell. The OGAB^{™} method can be suitably used for formation of the plasmid. In one embodiment, since strains such as Bacillus subtilis may have an ability to form a plasmid from a nucleic acid taken in from the outside, in the method, the nucleic acid to be introduced may not be a plasmid. For example, when being in contact with a nucleic acid (for example, an acyclic nucleic acid containing a tandem repeat nucleic acid sequence), Bacillus subtilis can take up the nucleic acid and form a plasmid within Bacillus subtilis (see, for example, the OGAB^{™} method described in the present specification). The construction of the construct may be referred to, for example, in WO 2022/097646 A.

### (Combinatorial library construction)

Combinatorial library construction will be described below. The following is exemplary, and other methods may also be used. A construct to which a promoter introduction site and a sequence for reconstruction are added is constructed. As described above, the OGAB^{™} method can be suitably used for construction of the plasmid. The sequence for reconstruction may be, for example, an SfiI, AarI, AlwNI, BbsI, BbvI, BcoDI, BfuAI, BglI, BsaI, BsaXI, BslI, BsmAI, BsmBI, BsmFI, BspMI, BspQI, BstAPI, BstXI, BtgZI, DraIII, EarI, Esp3I, FokI, HgaI, MwoI, PflMI, SfaNI, or SapI recognition sequence, and the like, and similarly, any sequence that generates an overhang of N upon cleavage can also be used. A seed plasmid for combinatorial library construction is constructed by introducing various promoters upstream of each gene in the gene cluster. A plurality of promoters having different transcription initiation timings and/or transcription strengths are selected. After performing combinatorial library construction, each construct in the library is introduced into a host cell, and one or more strains having the desired property are selected. Based on the selected strains, combinatorial library construction may be performed again. By performing multiple cycles of combinatorial library construction, transformation, and strain selection, strains having more excellent properties and/or multiple properties can be obtained (fine-tuning). For example, by fine-tuning the enzymes described in D. B. Basnet et al., J. Biotechnol., 2008, 135, 92-96 (EryCIII enzyme reaction, EryK/EryG enzyme reaction) and Appl. Microbiol. Biotechnol., 2017, 101, 5951, the production of the desired substance (the content ratio in the fraction) can be increased. The cycle may be performed two or more times, for example, two, three, four, or five times.

The selection of strains used for the 2nd cycle of combinatorial library construction may be performed according to any criterion. For example, strains ranking within the top five in terms of a desired property (for example, high production of a target substance) may be selected, or strains satisfying a predetermined property (for example, production of the target substance exceeding that of a natural strain) may be selected. The strains used for the 2nd cycle may be 2 or more, 50 or more, 100 or more, 1,000 or more, or 10,000 or more. Preferably, two or more strains are selected for use in the 2nd cycle. The strains used for the 2nd cycle may be the maximum number of transgenic organisms obtained, and may be, for example, 10, 15, 20, 30, 40, 50, or 100. The minimum and maximum numbers of strains to be selected may be any numbers between the above values.

### (Production of assembled nucleic acid)

Hereinafter, a procedure for producing an assembled nucleic acid to be incorporated into a transgenic organism will be described.

### Preparation of unit nucleic acid

A unit nucleic acid for incorporation into an assembled nucleic acid is prepared. The unit nucleic acid can be produced by any known method, for example, by polymerase chain reaction (PCR) or chemical synthesis. The unit nucleic acid may contain any desired sequence such as a sequence encoding a desired protein (such as a metabolic enzyme, a natural product biosynthetic enzyme group, an artificial enzyme, a therapeutic protein, and a protein constituting a viral vector) or a portion thereof, a sequence controlling a gene (such as promoter and an enhancer), or a sequence for manipulating a nucleic acid (such as a restriction enzyme recognition sequence). An end of each unit nucleic acid can be configured to produce a particular overhang sequence so that, when incorporated into an assembled nucleic acid, a plurality of types of unit nucleic acids are aligned in a particular order and/or orientation of property.

Since a large number of unit nucleic acids can ultimately be assembled onto a plasmid, one or a plurality of unit nucleic acids may be designed to encode one or a plurality of genes each having a long base length. Examples of genes having a long base length include, for example, a gene cluster constituting a series of metabolic pathways and a protein complex composed of a plurality of subunits.

### Preparation of unit vector

A unit vector can be prepared by linking a unit nucleic acid and an additional nucleic acid different from the unit nucleic acid. The use of the unit vector may allow for easier handling of the unit nucleic acid.

The additional nucleic acid may be a linear nucleic acid or a circular plasmid. When a circular plasmid is used as an additional nucleic acid, a unit vector can also have a circular structure, and thus can be used for transformation of, for example, E. coli or the like. In one embodiment, the additional nucleic acid can contain an origin of replication so that the unit vector is replicated in a target host. In one embodiment, all the unit nucleic acids for constructing a certain assembled nucleic acid may be linked to the same type of additional nucleic acid, thereby reducing a size difference between the unit vectors and facilitating the handling of a plurality of types of unit vectors. In one embodiment, a unit nucleic acid for constructing an assembled nucleic acid may be linked to different types of additional nucleic acids. In one embodiment, for one or a plurality of unit nucleic acids for constructing an assembled nucleic acid, a ratio of (base length of unit nucleic acid)/(base length of unit vector) or an average thereof can be 50% or less, 40% or less, 30% or less, 20% or less, 15% or less, 10% or less, 7% or less, 5% or less, 2% or less, 1.5% or less, 1% or less, or 0.5% or less. As the size of the additional nucleic acid is larger than the unit nucleic acid, the handling of different types of unit vectors can be more uniform. The linking between the unit nucleic acid and the additional nucleic acid can be performed by any method such as ligation using DNA ligase or TA cloning. In one embodiment, for one or a plurality of unit nucleic acids for constructing an assembled nucleic acid, a ratio of (base length of unit nucleic acid)/(base length of unit vector) or an average thereof can be 1% or more, 0.3% or more, 0.1% or more, 0.03% or more, 0.01% or more, 0.003% or more, or 0.001% or more, and the manipulation of the unit vector may be facilitated.

In one embodiment, the length of the unit nucleic acid may be 10 bp or more, 20 bp or more, 50 bp or more, 70 bp or more, 100 bp or more, 200 bp or more, 500 bp or more, 700 bp or more, 1,000 bp or more, or 1,500 bp or more and 5,000 bp or less, 5,000 bp or less, 2,000 bp or less, 1,500 bp or less, 1,200 bp or less, 1,000 bp or less, 700 bp or less, or 500 bp or less.

In one embodiment, the assembled nucleic acid can be constructed from 2 or more, 4 or more, 6 or more, 8 or more, 10 or more, 15 or more, 20 or more, 30 or more, 40 or more, 50 or more, 60 or more, 70 or more, 80 or more, 90 or more, or 100 or more, and 1,000 or less, 700 or less, 500 or less, 200 or less, 120 or less, 100 or less, 80 or less, 70 or less, 60 or less, or 50 or less types of unit nucleic acids. By adjusting the number of moles of each unit nucleic acid (or unit vector) to be substantially the same, a desired assembled nucleic acid having a tandem repeat-like structure can be efficiently produced.

In one embodiment, the unit nucleic acid may have a base length obtained by approximately equally dividing one set of repeat sequences in the assembled nucleic acid by the number of unit nucleic acids. This may facilitate a manipulation of aligning the number of moles of each unit nucleic acid (or unit vector). In one embodiment, the unit nucleic acid may have an increased or decreased base length of 30% or less, 25% or less, 20% or less, 15% or less, 10% or less, 7% or less, or 5% or less from a base length obtained by approximately equally dividing one set of repeat sequences in the assembled nucleic acid by the number of unit nucleic acids.

In one embodiment, the unit nucleic acid can be designed to have a non-palindromic sequence (a sequence that is not a palindromic sequence) at the end of the unit nucleic acid. The unit nucleic acid designed in this way can easily provide a structure in which the unit nucleic acids are linked to each other while maintaining the order in the assembled nucleic acid when the non-palindromic sequence is made into an overhang sequence.

### · Production of assembled nucleic acid

The assembled nucleic acid can be constructed by linking the unit nucleic acids to each other. In one embodiment, the unit nucleic acid can be prepared by being cut out from the unit vector by a restriction enzyme or the like. An assembled nucleic acid may contain one or more, two or more, three or more, four or more, five or more, six or more, seven or more, eight or more, nine or more, or ten or more sets of repeat sequences. The repeat sequence in the assembled nucleic acid may include a sequence of a unit nucleic acid and, as necessary, a sequence of an assembled vector nucleic acid. The assembled nucleic acid may contain a sequence that enables replication of a nucleic acid in a transgenic organism. In one embodiment, a sequence that allows replication of a nucleic acid in a transgenic organism can contain an effective origin of replication in a transgenic organism (for example, the genus Bacillus (Bacillus subtilis)). The sequence of the effective origin of replication in Bacillus subtilis is not particularly limited, and examples of a sequence having a θ-type replication mechanism include a sequence of an origin of replication contained in a plasmid such as pTB19 (Imanaka, T., et al. J. Gen. Microbioi. 130, 1399-1408 (1984)) or pLS32 (Tanaka, T and Ogra, M. FEBS Lett. 422, 243-246 (1998)), or pAMβ1 (Swinfield, T. J., et al. Gene 87, 79-90 (1990)).

The assembled nucleic acid may contain an additional base sequence, as necessary, in addition to the unit nucleic acid. In one embodiment, the assembled nucleic acid may contain a base sequence that controls transcription and translation, such as a promoter, an operator, an activator, or a terminator. Specific examples of a promoter in a case of using Bacillus subtilis as a host include Pspac (Yansura, D. and Henner, D. J. Pro. Natl. Acad. Sci, USA 81, 439-443 (1984)) whose expression can be controlled by isopropyl s-D-thiogalactopyranoside (IPTG), and a Pr promoter (Itaya, M. Biosci. Biotechnol. Biochem. 63, 602-604 (1999)).

The unit nucleic acid may form a repeat structure that maintains a certain order and orientation in the assembled nucleic acid. In one embodiment, the unit nucleic acid is constructed so that the base sequences at the overhang ends of the unit nucleic acid cut out from the unit vector are complementary to each other, such that it is possible to form a repeat structure in which a certain order and orientation in the assembled nucleic acid are maintained. In one embodiment, the structure of the overhang end is made unique for each different unit nucleic acid, such that it is possible to efficiently form a repeat structure maintaining a certain order and orientation. In one embodiment, the overhang ends may have a non-palindromic sequence and may be either a 5'-terminal overhang or a 3'-terminal overhang.

In one embodiment, a unit nucleic acid having an overhang end can be cut out from a unit vector using a restriction enzyme. In the embodiment, the unit vector may contain one or a plurality of restriction enzyme recognition sequences. In a case where the unit vector contains a plurality of restriction enzyme recognition sequences, each restriction enzyme recognition sequence may be recognized by the same restriction enzyme or may be recognized by different restriction enzymes. In one embodiment, the unit vector can contain a pair of regions recognized by the same restriction enzyme so that a complete unit nucleic acid region is included between these regions. In an embodiment using a restriction enzyme that cleaves a recognition region, the unit vector may contain a region recognized by the restriction enzyme at the end of the unit nucleic acid region.

In one embodiment, when the same type of restriction enzyme is used to cut out the unit nucleic acid from a plurality of unit vectors, the restriction enzyme treatment can be performed in a solution containing the plurality of unit vectors, and the efficiency of the operation can be improved. The types of restriction enzymes used to produce a certain assembled nucleic acid may be, for example, 5 or less, 4 or less, 3 or less, 2 or less, or 1, and the use of a small number of restriction enzymes can reduce a variation in the number of moles between unit nucleic acids. In one embodiment, the unit nucleic acid cut out from the unit vector can be readily purified by any known fractionation method such as agarose gel electrophoresis.

The unit nucleic acids and, as necessary, the assembled vector nucleic acids can be linked (ligated) to each other using DNA ligase or the like. Therefore, an assembled nucleic acid can be produced. For example, linking of unit nucleic acids, and as necessary, assembled vector nucleic acids can be performed in the presence of components such as polyethylene glycol (for example, PEG2000, PEG4000, PEG6000, PEG8000, or the like) and a salt (for example, monovalent alkali metal, sodium chloride, or the like). A concentration of each unit nucleic acid in a ligation reaction solution is not particularly limited, and may be 1 fmol/µl or more. A reaction temperature and time of the ligation are not particularly limited, and are, for example, 30 minutes or longer at 37°C. In one embodiment, prior to ligating the unit nucleic acid and, as necessary, the assembled vector nucleic acid, the composition containing the unit nucleic acid and, as necessary, the assembled vector nucleic acid may be subjected to any conditions that inactivate a restriction enzyme (for example, phenol and chloroform treatment).

The unit nucleic acid can be adjusted to about the same number of moles using a method described in WO 2015/111248 A or the like. By adjusting the unit nucleic acid to substantially the same number of moles, a desired assembled nucleic acid having a tandem repeat-like structure can be efficiently produced. The number of moles of the unit nucleic acid can be adjusted by measuring the concentration of the unit vector or the unit nucleic acid.

### · Production of plasmid using assembled nucleic acid

Bringing the assembled nucleic acid into contact with a transgenic organism can form a plasmid in the transgenic organism. In one embodiment, examples of the transgenic organism include the genus Bacillus, the genus Streptococcus, the genus Haemophilus, and the genus Neisseria. Examples of the genus Bacillus include B. subtilis (Bacillus subtilis), B. megaterium (Bacillus megaterium), and B. stearothermophilus (Bacillus stearothermophilus), and any transgenic organism capable of forming a plasmid from an assembled nucleic acid can be used. In one embodiment, the transgenic organism that incorporates the assembled nucleic acid is in a competent state and is able to actively incorporate the nucleic acid. For example, Bacillus subtilis in a competent state cleaves a double-stranded nucleic acid as a substrate on a cell, decomposes any one strand of the double-stranded nucleic acid from the cleavage point, and incorporates the other single strand into a bacterial cell. The incorporated single strand can be repaired into a circular double-stranded nucleic acid in the bacterial cell. While any known method can be used to render a transgenic organism competent, for example, Bacillus subtilis can be rendered competent using the methods described in Anagnostopoulou, C. and Spizizen, J. J. Bacteriol., 81, 741-46 (1961). As a method of transformation, a known method suitable for each transgenic organism can be used.

In one embodiment, the present disclosure provides a vector plasmid produced from a packaging cell. In one embodiment, a vector plasmid produced from a packaging cell can be purified using any known method, and the present disclosure also provides a vector plasmid thus purified. In one embodiment, it can be confirmed that the purified vector plasmid contains a desired nucleic acid sequence by examining the size pattern of fragments generated by restriction enzyme cleavage, a PCR method, a base sequencing method, or the like. In one embodiment, a composition containing the vector plasmid prepared by the vector plasmid production method of the present disclosure may contain a small amount of endotoxin. In one embodiment, there is provided Bacillus subtilis containing a vector plasmid of the present disclosure.

In the present disclosure, the transgenic organism (host cell) that is used may be selected from Bacillus subtilis, actinomycetes, filamentous fungi, yeast, hydrogen bacteria, coryneform bacteria, or animal cells. A preferred transgenic organism is Bacillus subtilis. It is preferable that these transgenic organisms are capable of generating a plasmid from the assembled nucleic acid. The transgenic organism for generating a plasmid from the assembled nucleic acid and performing combinatorial library construction, and the transgenic organism for producing a substance, may be the same or may be different. For example, as the transgenic organism for combinatorial library construction, Bacillus subtilis may be used, and as the transgenic organism for producing a substance, the same Bacillus subtilis may be used, or another transgenic organism, such as an actinomycete, a filamentous fungus, yeast, a hydrogen bacterium, a coryneform bacterium, or an animal cell may be used.

In one embodiment, the undesired restriction enzyme recognition sequence may be a restriction enzyme recognition sequence used in the OGAB^{™} method or the Combi-OGAB^{™} method, and may be, for example, a Type IIS restriction enzyme recognition sequence selected from the group consisting of AarI, BsaI, BbsI, BsmBI, and BspQI, and a reconstruction sequence such as an SfiI recognition sequence.

In one embodiment, the gene group related to substance production may be a gene cluster of polyketide synthases (PKSs), non-ribosomal peptide synthases (NRPSs), ribosomally synthesized and post-translationally modified peptides (RiPPs) biosynthetic enzymes, or terpene biosynthetic enzymes.

Examples of the combination of the promoters in the construct of the strain having the desired property in the present disclosure include, but are not limited to, any combination selected from PabrB, PvalS, Phag, PspoVG, PyvyD, PhemA, Pffh, PsdhB, PiolS, Pspo0A, PglyA, PyceC, PsrfAA, PyrxA, PytxG, PytvI, PspoVB, PphrK, PphrF, PftsH, Pasd, Pspo0F, PmenB, PgltX, PminC, PphrC, PspoVS, PlicH, Pcdd, PflgM, PcheV, PcitZ, PclpQ, PphrG, PlytR, Phbs, PodhA, PsecA, PphrE, PftsZ, PxpaC, PcsbA, PywjC, PyknW, PysdB, PrapI, PyteJ, PureA, PtrxA, Pdps, PxynA, PrsbW, PspoIVCA, PyqfY, PyqeZ, PyabR, PkinA, PnadE, PsigH, PresE, PspsA, PgspA, PyflG, Pveg, Pgap, PahpC, PyvrE, PytkL, PyfkJ, PdegS, PpyrAB, PyacL, PywkA, PspoVT, PywtG, PpdhC, PyuzA, PyhdF, PalsD, PpurE, PythP, PcomK, PptsI, PgtaB, PkatX, PydbP, PyqgZ, PydjO, PlytE, PgsiB, PywrE, PopuE, PyoxA, PthrS, PbltD, PyfhL, PyraD, PydaD, PywnJ, PrelA, PydfK, PgerBC, PyitG, PybfO, PcotH, PylnF, PribT, PrapH, PmmgA, PyqfD, and PsigW, and any other growth-phase-dependent promoters of Bacillus subtilis may be appropriately used.

In the present specification, "or" is used when "at least one or more" of the items listed in the text can be employed. The same applies to "or". When explicitly described in the present specification as "within the range" of "two values", the range also includes the two values themselves.

References such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present specification, but is limited only by the claims.

### Examples

The present disclosure will be described in detail by the following Examples; however, the present disclosure is not limited to these Examples. For reagents, the specific products described in the examples were used. However, the reagent can be substituted with an equivalent product from another manufacturer (Sigma-Aldrich, Wako Pure Chemical, NACALAI TESQUE, INC., R&D Systems, USCN Life Science INC, or the like).

### <Reagents and test methods>

The reagents and common test methods used in the Examples are as follows.

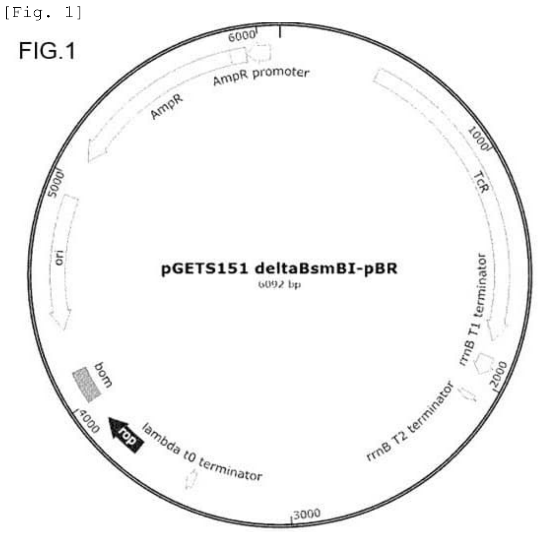

As host Bacillus subtilis strains, the Marburg 168 strain and the BUSY9797 strain, which is a derivative of the RM125 strain (Non Patent Literature 7), were used. As a plasmid vector capable of replicating in Bacillus subtilis, a variant of pGETS151-pBR (Patent Literature 2) (FIG. 1, SEQ ID NO: 1) was used. As the antibiotics carbenicillin, tetracycline, and kanamycin, those manufactured by NACALAI TESQUE, INC. were used. Lysozyme was purchased from FUJIFILM Wako Pure Chemical Corporation. Each restriction enzyme was purchased from New England Biolabs, Inc. T4 DNA ligase was purchased from Takara Bio Inc. General ligation for construction of plasmids in E. coli was performed using the Takara Ligation Kit <Mighty Mix> (Takara Bio Inc.). PCR reactions for preparing unit DNAs were performed using KOD Plus polymerase v.2 from Toyobo Co., Ltd. On the other hand, for colony PCR for sequencing DNA cloned into plasmids, Ex-Taq HS manufactured by Takara Bio Inc. was used. As the enzyme Plasmid Safe for purification of a circular plasmid, an enzyme manufactured by EPICENTER was used. 2-Hydroxyethyl agarose, which is low-melting-point agarose gel for DNA electrophoresis, was purchased from Sigma. As another common agarose for electrophoresis, UltraPure Agarose from Invitrogen was used. TE-saturated phenol (containing 8-xylinol) was purchased from NACALAI TESQUE, INC. As the LB medium components and agar, a medium manufactured by Becton, Dickinson and Company was used. Each of the reagents used for extraction from cells or culture media after culture, and for analysis by HPLC, was manufactured by NACALAI TESQUE, INC.

E. coli JM109 strain was used for plasmid construction. For small-scale purification of the constructed plasmid from E. coli, the QIA prep Spin Miniprep Kit from Qiagen was used. For purification of DNA from the enzyme reaction solution, the MinElute Reaction Cleanup Kit from Qiagen was used. The NanoDrop One from Thermo was used as an ultramicro spectrophotometer. For determination of base sequences, a 3500xL Genetic Analyzer manufactured by Applied Biosystems, which is a fluorescence-based automatic sequencer, was used.

Other general DNA manipulations were performed according to standard protocols (Non Patent Literature 8). Transformation of Bacillus subtilis and plasmid extraction by the OGAB^{™} method and the like were performed as previously reported (Non Patent Literature 9).

### 1. Construction of plasmid vector for assembly

The plasmid vector pGETS151-pBR for assembly is an E. coli-Bacillus subtilis shuttle plasmid vector having the origin of replication of E. coli pBR322 and the origin of replication of Bacillus subtilis pGETS151. For this vector, a promoter was introduced to increase the plasmid copy number, and a point mutation was introduced into the BsmBI recognition sequence contained in pGETS151 so that the site was not cleaved. The vector thus constructed was used in the present Example. The structure of the vector is shown in FIG. 1, and the base sequence thereof is shown in SEQ ID NO: 1. The present plasmid vector was cleaved with the restriction enzyme AarI and purified to obtain a pGETS151 vector in which the pBR322-derived sequence was removed and protruding ends ATTA and AAAA were generated. Using these protruding ends, the desired gene cluster was introduced, and a Bacillus subtilis plasmid was constructed.

### 2. Selection of natural product biosynthetic enzyme gene cluster

As the biosynthetic gene clusters (BGCs) addressed in the Examples of the present disclosure, the Triketide Pyrone BGC (bpsA-bpsB), which is a Type III PKS contained in the genome of Bacillus subtilis, and the Gramicidin S BGC (grsT-grsA-grsB), which is an NRPS contained in the genome of Aneurinibacillus migulanus DSM2895, were targeted. The production of the two substances addressed herein is not detected under ordinary conditions in wild-type strains (Non Patent Literatures 10 and 11). In the Examples shown below, the Triketide Pyrone BGC was produced in Bacillus subtilis Marburg 168, which naturally possesses the BGC in its genome (homologous production), and the Gramicidin S BGC was examined in Bacillus subtilis BUSY9797, which does not possess the BGC in its genome (heterologous production). In the present study, the most important task was set as creating a strain capable of producing, in the same species or in a different species, a substance that is not produced, or is produced only in an extremely small amount, in a wild-type host. For this purpose, combinatorial library construction of growth-phase-dependent promoters having different transcription strengths was used, on a gene-by-gene basis for the genes constituting the gene clusters.

### Example 1

### (1) Preparation of seed plasmid for BGC-1 (Triketide Pyrone BGC)

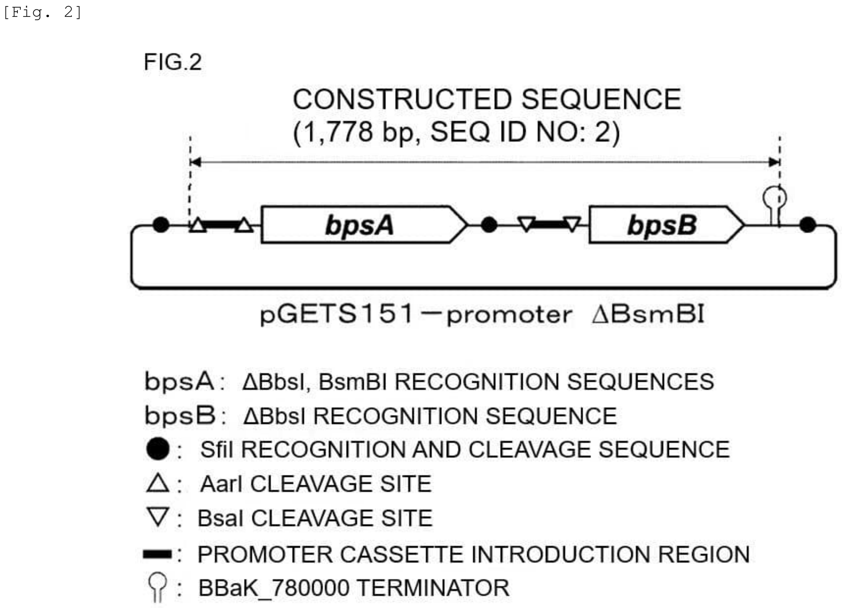

In BGC-1, the Triketide pyrone core is produced by BpsA, which is a Type III PKS, and the OH group on the core is further methylated by BpsB, which is a methyltransferase. An attempt was made to obtain a strain that highly produces this methylated Triketide Pyrone (Pyrone-OMe), and a strain that reduces the residual amount of unmethylated Triketide Pyrone (Pyrone-OH) produced by the intermediate BpsA. From the bpsA and bpsB sequences of Bacillus subtilis, sequences in which the recognition sites of BbsI and BsmBI were silenced were designed, and 19 bp upstream of bpsA and 15 bp upstream of bpsB on the wild-type genome were added upstream of each gene with the expectation that the Shine-Dalgarno (SD) sequence was contained. Furthermore, a promoter introduction site and an SfiI recognition sequence for Combi-OGAB^{™} were introduced (SEQ ID NO: 2). After this sequence was constructed by the MAP method (Patent Literature 3), it was placed on a vector for Bacillus subtilis (FIG. 2). For this construct, a cassette in which various promoters were added downstream of the BBaK_780000 terminator was introduced (Table 1). As the promoters, growth-phase-dependent promoters described in Non Patent Literature 6 were used. As the growth-phase-dependent promoters, the following were used: promoters in which transcription starts in the logarithmic growth phase (Class I), PabrB (transcription strength: strong), PvalS (transcription strength: medium), and Phag (transcription strength: weak); promoters in which transcription starts from the mid-logarithmic growth phase to the early stationary phase (Class II), PspoVG (transcription strength: strong), PsrfAA (transcription strength: medium), and Pcdd (transcription strength: weak); promoters in which transcription starts in the lag phase and the stationary phase (Class III), PlytR (transcription strength: strong) and Pveg (transcription strength: weak); and promoters in which transcription starts in the stationary phase (Class IV), PmmgA (transcription strength: strong), PyqfD (transcription strength: medium), and PsigW (transcription strength: weak). Plasmids into which these promoter cassettes were introduced in both bpsA and bpsB were first prepared and used as seed plasmids. Next, using these 11 types of seed plasmids, combinatorial library construction was performed by the Combi-OGAB^{™} method, and a search was conducted for combinations of promoters with optimal transcription strength and transcription initiation timing in order to obtain a strain that highly produces Pyrone-OMe and a strain that can suppress the residual level of Pyrone-OH.

**[Table 1]**

| Promoter cassette name | Sequence |
|---|---|
| BBaK_780000-PabrB | AAGAAGAAGTGTGAAAAAGCGCAGCTGAAATAGCTGCGCTTTTTTGTGTCATAATCCTAATATATACATACGCCCTGAAAAAGA ATAATTAAAATTAAAAATCTTTAATGAAAATAAAAAAAGTCTGTTCGTGTCGAATTTTGTCGTATATAATCAAATGAAATAAAAA ACTTTGGTTCGAAAGTCTTGATTTAAAAGGATTTTTAGTAGGATAATAGCTTTATTAAATATTTATAAAATGCTGTTATTTCGGT AGTTTCCAAGACATTACTGACTATAAGAACTAATTCTTACAATCAATAGTAAACAAAATGATTGACGATTATTGGAAACCTTGTT ATGCTATGAAGGTAAGGAT (SEQ ID NO: 4) |
| BBaK_780000-PvalS | AAGAAGAAGTGTGAAAAAGCGCAGCTGAAATAGCTGCGCTTTTTTGTGTCATAATCCTAGATTAGGATTGCGAGCAGCAAAAC ATCAAAGGTTGTCGGAAAGAAAATGGTTCTGATTCCTTGAAAAATCGTAATGGGAATAATCACTTGTGCGGATACAAGCCGAA TTTGCCTTACCCATGGCGGCAGCGAATAAGAGCTGAAACGTTTCTTCACGGTGCATCCTCCTCTACGCATTTACAAGCATCAT ATGTAAACTTGGGCAATAGGTGCCTGCCCATCTGTATAGAAAGCGGTGTTTTTGGAAAAGATGATTGACGAATCAAAAACACT TTTACTATAATAAAGAACATAAATAA (SEQ ID NO: 5) |
| BBaK_780000-Phag | AAGAAGAAGTGTGAAAAAGCGCAGCTGAAATAGCTGCGCTTTTTTGTGTCATAATCCTTGAAGTAAAAGTGATTGCGGTTGAA GGGGATCAAGTGAAGCTTGGAATTGACGCCCCAAAGCATATTGATATTCACAGGAAAGAAATTTACTTGACCATTCAGGAAGA AAATAACCGTGCAGCAGCGTTATCCAGCGATGTGATCTCCGCATTATCCTCACAAAAAAAGTGAGGATTTTTTTATTTTTGTAT TAACAAAATCAGAGACAATCCGATATTAATGATGTAGCCGGGAGGAGGCGCAAAAGACTCAGCCAGTTACAAAATAAGGGCAC AAGGACGTGCCTTAACAACATATTC (SEQ ID NO: 6) |
| BBaK_780000-PspoVG | AAGAAGAAGTGTGAAAAAGCGCAGCTGAAATAGCTGCGCTTTTTTGTGTCATAATCCTTGCGGAAGTAAACGAAGTGTACGGA CAATATTTTGACACTCACAAACCGGCGAGATCTTGTGTTGAAGTCGCGAGACTCCCGAAGGATGCGTTAGTCGAGATCGAAGT TATTGCACTGGTGAAATAATAAGAAAAGTGATTCTGGGAGAGCCGGGATCACTTTTTTATTTACCTTATGCCCGAAATGAAAGC TTTATGACCTAATTGTGTAACTATATCCTATTTTTTCAAAAAATATTTTAAAAACGAGCAGGATTTCAGAAAAAATCGTGGAATT GATACACTAATGCTTTTATATAG (SEQ ID NO: 7) |
| BBaK_780000-PsrfAA | AAGAAGAAGTGTGAAAAAGCGCAGCTGAAATAGCTGCGCTTTTTTGTGTCATAATCCTGACGCTCTTCGCAAGGGTGTCTTTT TTTGCCTTTTTTTCGGTTTTTGCGCGGTACACATAGTCATGTAAAGATTGTAAATTGCATTCAGCAATAAAAAAAGATTGAACG CAGCAGTTTGGTTTAAAAATTTTTATTTTTCTGTAAATAATGTTTAGTGGAAATGATTGCGGCATCCCGCAAAAAATATTGCTGT AAATAAACTGGAATCTTTCGGCATCCCGCATGAAACTTTTCACCCATTTTTCGGTGATAAAAACATTTTTTTCATTTAAACTGAA CGGTAGAAAGATAAAAAATAT (SEQ ID NO: 8) |
| BBaK_780000-Pcdd | AAGAAGAAGTGTGAAAAAGCGCAGCTGAAATAGCTGCGCTTTTTTGTGTCATAATCCTTATTGAGTGGATGATTATATTCCTTT TGATAGGTGGTATGTTTTCGCTTGAACTTTTAAATACAGCCATTGAACATACGGTTGATTTAATAACTGACAAACATCACCCTC TTGCTAAAGCGGCCAAGGACGCTGCCGCCGGGGCTGTTTGCGTTTTTGCCGTGATTTCGTGTATCATTGGTTTACTTATTTTT TTGCCAAAGCTGTAATGGCTGAAAATTCTTACATTTATTTTACATTTTTAGAAATGGGCGTGAAAAAAAGCGCGCGATTATGTA AAATATAAAGTGATAGCGGTACC (SEQ ID NO: 9) |
| BBaK_780000-PlytR | AAGAAGAAGTGTGAAAAAGCGCAGCTGAAATAGCTGCGCTTTTTTGTGTCATAATCCTCTAACCCTACATAAGTACCTTCTTTT GTTTCAATGTTACTGTCTGGCGATACATCTTCACCTTGACTCTTTTGACTATTAACCCCGCAACCCGAAAGAAGCAATATAAAG AACAGTAAAGCAATAAATTTTTTCATTTTTTTCACCTCATTATATTTTATCGTCAACCTATTTTATATTTTAAAGAAAAATTAAGA AACAATGAAACTTTTTTTTATAAAAAACGACTATTTTAGGATTTCATTCTTGTATTAAATAGAGTTGTATTTATTGGAAATTTAAC TCATAATGAAAGTAATTT (SEQ ID NO: 10) |
| BBaK_780000-Pveg | AAGAAGAAGTGTGAAAAAGCGCAGCTGAAATAGCTGCGCTTTTTTGTGTCATAATCCTTATGGGAAGTGCTCCGTAATACGCT GACAAGAGAGAAAGGGCTTGGAGGTATTGAAACAAGAGGAGTTCTGAGAATTGGTATGCCTTATAAGTCCAATTAACAGTTGA AAACCTGCATAGGAGAGCTATGCGGGTTTTTTATTTTACATAATGATACATAATTTACCGAAACTTGCGGAACATAATTGAGGA ATCATAGAATTTTGTCAAAATAATTTTATTGACAACGTCTTATTAACGTTGATATAATTTAAATTTTATTTGACAAAAATGGGCT CGTGTTGTACAATAAATGTAGTG (SEQ ID NO: 11) |
| BBaK_780000-PmmgA | AAGAAGAAGTGTGAAAAAGCGCAGCTGAAATAGCTGCGCTTTTTTGTGTCATAATCCTTCGATATATCGCGTCTATTCCGGCTT CCGGCTATCACCCGAAGATAAACAGCCCAGGGGTCACAGATGAAGTACTGAAGAAAATGAGGAACGGTTTGATTAAGGTAAG GCCGTATACAGTCAATCGTCCGGAAGATATGAAGCGTCTCATTGAAGCGGGAGCAGACGGCATGTTTACCGACTTTCCAGAAA AGGCTTCGGCATTGCTGAAAAATGAATAGTTGTTAGAAGGAGGCTGTTTGACGCAGCCTTCTTTTTTCATTCATTCATGCCCGT TTCAAAGCATACATTCATAGAAGAC (SEQ ID NO: 12) |
| BBaK_780000-PyqfD | AAGAAGAAGTGTGAAAAAGCGCAGCTGAAATAGCTGCGCTTTTTTGTGTCATAATCCTAGATGCCCCTGCACCTATCCCTAAC CGTATGGAACAGGCAAGACGGGAAGCGGAAGAAAGACGCAGGGAAACAGCAAGAAACCTGAAAGGGCTGGAACGAGATCTTG CTGCTGCCAAACAAAAAACAGTATACACAAAACAAAAAATGCTTCAGGTGAATAAAGACACCGTCGTACAGGGGATCGTTCTA GGAGAGGTGTTCGGACCTCCACGGGCGAAAAAACCTCACCGTACGATGCGCCCGGCCCGTAAAAATTAAAGTGTTAGAACCT CCTTTCAAATCATACATATGAGATGAAA (SEQ ID NO: 13) |
| BBaK_780000-PsigW | AAGAAGAAGTGTGAAAAAGCGCAGCTGAAATAGCTGCGCTTTTTTGTGTCATAATCCTTGCCCCCCTCCACCATTATTGGGCT ATAGCCAAGCGGTAAGGCAACGGACTTTGACTCCGTCATGCGTTGGTTCGAATCCAGCTAGCCCAGTCACAGACACCTTTGAT CAAAAGGTGTCTTTTTTCTTTTCGGAAAAATCATTCCAACTTCTAACTGTTCAGTCTGTATAATAATTTTAAAAATATGTTAAGG TAGTTTATTCACGAATTACCATCTACACCCTGCCAAAAATTTGATAAACTTATTTTATAAAAAAATTGAAACCTTTTGAAACGAA GCTCGTATACATACAGACCGGT (SEQ ID NO: 14) |

### (2) Screening of high-methylated Triketide Pyrone-producing strains by Combi-OGAB^{™}

Equal amounts of the 11 types of plasmids were mixed, and after cleavage with SfiI, the enzymes were inactivated with TE-saturated phenol, the aqueous phase was concentrated with 1-butanol, ethanol precipitation was performed, and the precipitate was dissolved in TE. This SfiI-treated fragment solution was again ligated with T4 DNA ligase, and after being assembled in the Marburg 168 strain, plating was performed on LB plates containing 10 µg/mL tetracycline, and incubation was performed overnight at 30°C. Colonies that appeared were each inoculated into 300 µL of LB liquid medium containing 10 µg/mL tetracycline, cultured overnight at 30°C, and then stored frozen at -70°C. For evaluation of the Triketide Pyrone biosynthetic amount of each strain, inoculation was performed from the frozen stock into 10 mL of LB liquid medium containing 10 µg/mL tetracycline, and shaking culture at 37°C (17 hours in 1st cycle, 24 hours in 2nd to 4th cycles) was performed. The culture medium was centrifuged at 8,000 g for 5 minutes, and the precipitate was collected. Next, the precipitate was suspended in 15 mL of pure water and re-centrifuged. The precipitate was suspended in 5 mL of 150 mM NaCl and 20 mM HCl and incubated at 80°C for 30 minutes, 5 mL of ethyl acetate was added, and the mixture was stirred with a vortex mixer for 30 seconds. Thereafter, the mixture was centrifuged at 10,000 g for 10 minutes, and the organic layer was collected. The collected organic layer was dried using an evaporator and dissolved in 100 µL of methanol. The solution was centrifuged at 15,000 g for 5 minutes, and 20 µL of the supernatant was injected into HPLC to analyze the production of Triketide Pyrone. The HPLC measurement conditions are as follows.
Column: COSMOSIL (trademark) 5C18-MS-II Packed Column, 4.6 mm I.D. x 150 mm
Mobile phase A: 0.1% formic acid in pure water, Mobile phase B: methanol
Gradient: (80% B, 3 minutes) - (80% → 98% B, 4 minutes) - (98% B, 18 minutes)
Flow rate: 1 mL/min
Detection: 280 nm

After completion of the analysis, a plurality of strains that highly produced Pyrone-OMe as the target product were selected from the obtained data, and each strain was cultured. The resulting overnight culture media were mixed in equal amounts, and the plasmids were extracted. Subsequently, the cycle of SfiI treatment, ligation, transformation of Marburg 168, and analysis was performed until the production converged. Note that, among the Triketide Pyrone derivatives that appeared in the present study, the peak at m/z 350 was assigned to Pyrone-OMe, and the peak at m/z 336 was assigned to Pyrone-OH for quantification.

### (3) Transition of proportion of Pyrone-OMe high-producing strains in each Combi-OGAB^{™} cycle

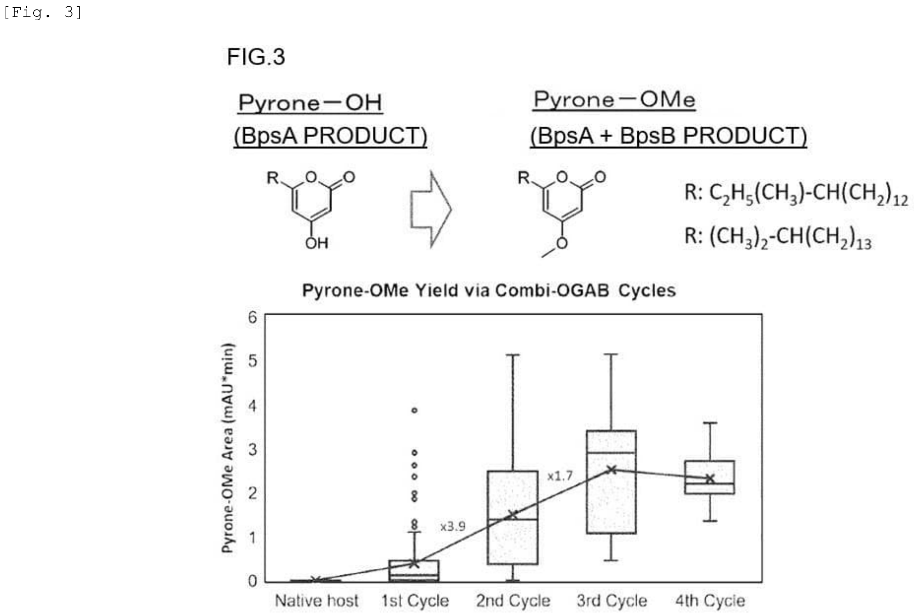

In the 1st cycle, when 144 strains were individually cultured, 123 strains grew. Extracts were prepared from those strains by the above method, and after HPLC analysis, a 2nd cycle library was constructed using 13 high-producing strains. From the 2nd cycle onward, all the strains grew (Table 2). The production of Pyrone-OMe, which is the final product, reached the upper limit in the 3rd cycle (FIG. 3). When the promoter sequences of the top three high-producing strains for the target substance were measured in the 2nd and 3rd cycles, three out of the six strains had the combination PvalS-bpsA-PsrfAA-bpsB (Table 3). That is, in order to construct a Bacillus subtilis strain that highly produces Pyrone-OMe, the combination of promoters PvalS (Class I-medium) and PsrfAA (Class II-medium) is optimal, and it was demonstrated that Pyrone-OMe can be efficiently obtained by using this strain.

**[Table 2]**

| | 1st cycle | 2nd cycle | 3rd cycle | 4th cycle |
|---|---|---|---|---|
| Number of seed plasmid strains | - | 13 | 9 | 6 |
| Number of cultured strains | 144 | 60 | 30 | 20 |
| Culture time (hr) | 17 | 24 | 24 | 24 |
| Growth count (= HPLC analysis count) | 123 | 60 | 30 | 20 |

**[Table 3]**

| | 2nd cycle | 3rd cycle |
|---|---|---|
| Highest-production strain | 1 D6 | A1 strain |
| | Area value =5. 1297 | Area value =5. 1454 |
| | Promoter combination: PabrB-PvalS | Promoter combination: PvalS - PsrfAA |
| Second-highest-production strain | 1E12 strain | A7 strain |
| | Area value =4. 567 | Area value =5. 0566 |
| | Promoter combination : PvalS - PsrfAA | Promoter combination : PvalS-PsrfAA |
| Third-highest-production strain | 1D7 strain | B6 strain |
| | Area value =3. 6827 | Area value =4. 6486 |
| | Promoter combination : PlytR-Phag | Promoter combination: PvalS-PspoVG |

### (4) Transition of proportion of strains with high methylation efficiency in each Combi-OGAB^{™} cycle

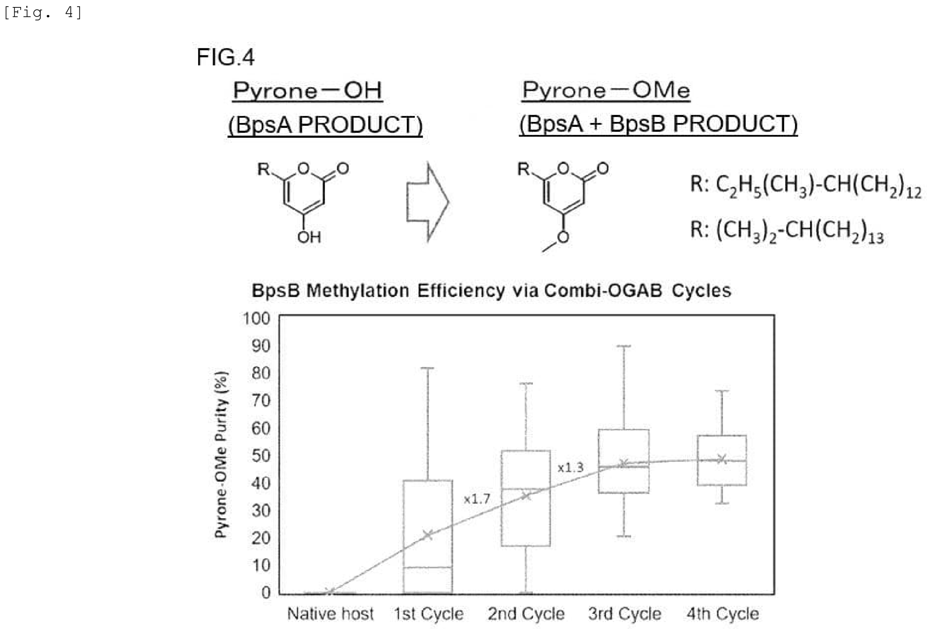

When the production of Pyrone-OMe was monitored for each cycle, samples were also observed in which Pyrone-OMe occupied a high proportion relative to the total amount of the unmethylated form (Pyrone-OH), which is produced by BpsA and not methylated by BpsB, and Pyrone-OMe. Therefore, the transition in each cycle based on the above production was reanalyzed using the methylation rate by BpsB (100 x Pyrone-OMe production/(Pyrone-OH production + Pyrone-OMe production)). This also increased with each cycle and converged in the 3rd cycle (FIG. 4). The combination that provided the highest methylation efficiency in the 3rd cycle was Pcdd-bpsA-PvalS-bpsB (Table 4). That is, it was concluded that, in order to construct a Bacillus subtilis strain that efficiently methylates Pyrone-OH, the optimal combination of promoters is Pcdd (Class II-weak) and PvalS (Class I-medium). Although the production of Pyrone-OMe with this promoter combination was not as high as that with the PvalS-PsrfAA combination described above, it was demonstrated that Pyrone-OH and Pyrone-OMe, which are detected at closely eluting times on HPLC, can be efficiently and conveniently separated.

**[Table 4]**

| | 2nd cycle | 3rd cycle |
|---|---|---|
| Highest-production strain | 1 D6 | B12 strain |
| | Purity =76% | Purity =90% |
| | Promoter combination: PabrB-PvalS | Promoter combination : Pcdd-PvalS |
| Second-highest-production strain | 1D12 strain | A1 strain |
| | Purity =75% | Purity =69% |
| | Promoter combination : PlytR-PsrfAA | Promoter combination: PvalS-PsrfAA |
| Third-highest-production strain | 1E12 strain | B6 strain |
| | Purity=70% | Purity =66% |
| | Promoter combination : PvalS-PsrfAA | Promoter combination: PvalS-PspoVG |

### Example 2

### (1) Preparation of seed plasmids for BGC-2 (Gramicidin S BGC)

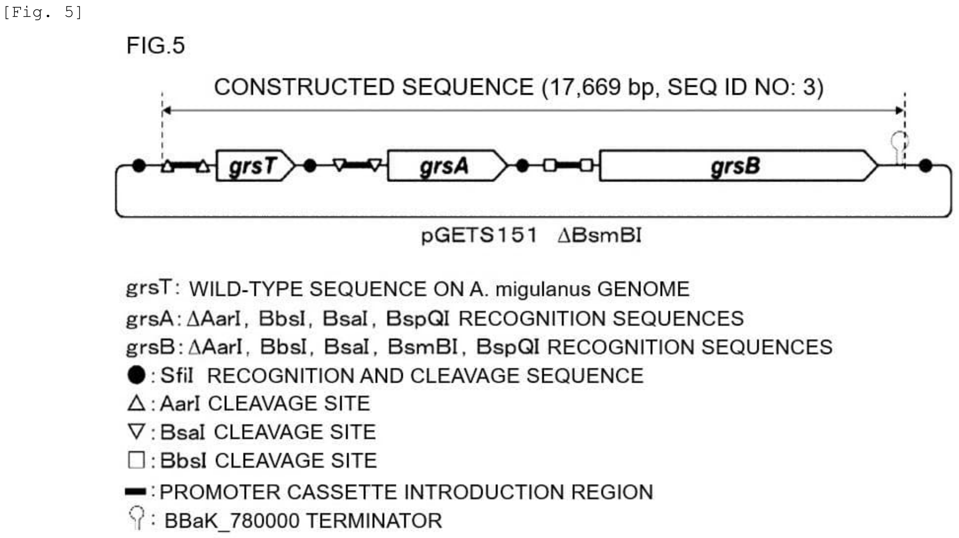

Similar to BGC-1, by searching for promoters having suitable transcription strength and transcription initiation timing for grsT, grsA, and grsB, which constitute the Gramicidin S BGC, the optimal combination of promoters for highest production of Gramicidin S was examined. From the grsT, grsA, and grsB sequences of the DSM 2895 strain, sequences in which the recognition sites of AarI, BsaI, BbsI, and BspQI were silenced were designed, and 20 bp upstream of grsT, 22 bp upstream of grsA, and 20 bp upstream of grsB of the DSM 2895 strain were added upstream of each gene with the expectation that the SD sequence was contained. In addition, similar to BGC-1, promoter introduction sites and SfiI recognition sequences for Combi-OGAB^{™} were introduced (SEQ ID NO: 3). This sequence was divided into 25 OGAB^{™} blocks and synthesized by the OGAB^{™} method, and then assembled in a vector for Bacillus subtilis (FIG. 5). Thereafter, as in BGC-1 described above, a plasmid in which a cassette linking any one of the promoters PvalS (Class I-medium), Phag (Class I-weak), PspoVG (Class II-strong), PsrfAA (Class II-medium), Pcdd (Class II-weak), PlytR (Class III-strong), Pveg (Class III-weak), PmmgA (Class IV-strong), PyqfD (Class IV-medium), and PsigW (Class IV-weak) at the promoter introduction site was introduced downstream of the BBaK_780000 terminator into all of grsT, grsA, and grsB was prepared as a seed plasmid. Next, using these 10 types of seed plasmids, combinatorial library construction was performed by the Combi-OGAB^{™} method, and a search was conducted for combinations of promoters with optimal transcription strength and transcription initiation timing in order to obtain a strain that highly produces Gramicidin S.

### (2) Screening of Gramicidin S high-producing strains by Combi-OGAB^{™}

Equal amounts of the 10 types of plasmids were mixed, and after cleavage with SfiI, the enzyme was inactivated with TE-saturated phenol, the aqueous phase was then concentrated with 1-butanol, ethanol precipitation was performed, and the resulting precipitate was dissolved in TE. This SfiI-treated fragment solution was again ligated with T4 DNA ligase, and after assembly in a BUSY9797 strain into which plasmid pUB8 (Non Patent Literature 12), encoding the phosphopantetheinyl transferase lpa-8, was introduced, the assembled product was plated on LB plates containing 10 µg/mL tetracycline and kanamycin and incubated overnight at 30°C. Colonies that appeared were each inoculated into 300 µL of LB liquid medium containing 10 µg/mL tetracycline and kanamycin, cultured overnight at 30°C, and then stored frozen at -70°C. For culture to evaluate the Gramicidin S biosynthesis level, inoculation was performed from the frozen stock into 2 mL of YTG (Bacto Yeast Extract 50 g/L, Bacto Tryptone 50 g/L, glucose 5 g/L) liquid medium containing 10 µg/mL tetracycline and kanamycin, and shaking culture was performed at 30°C for 72 hours. To the culture medium, 2 mL of ethyl acetate was added, the mixture was stirred with a vortex mixer for 30 seconds and centrifuged at 10,000 g for 10 minutes, and the organic layer was collected. The collected organic layer was dried using an evaporator and dissolved in 200 µL of 70% methanol-0.5% formic acid. The solution was centrifuged at 15,000 g for 5 minutes, and 20 µL of the supernatant was injected into HPLC to analyze the production of Gramicidin S. The HPLC measurement conditions are as follows.
Column: COSMOSIL (trademark) 5C18-AR-II Packed Column, 4.6 mm I.D. x 150 mm
Mobile phase A: 0.1% formic acid in pure water, Mobile phase B: methanol
Gradient: (60% B, 5 minutes) - (60% → 78% B, 9 minutes) - (78% 100% B, 0.1 minutes) - (100% B, 5 minutes)
Flow rate: 1 mL/min
Detection: 210 nm

After completion of the analysis, a plurality of high-producing strains were selected from the obtained data, and after re-culture, the resulting culture media were mixed in equal amounts, and plasmids were extracted. Thereafter, SfiI treatment was performed to reconstruct the library, and the Combi-OGAB^{™} cycle, in which the production of each strain was similarly measured, was repeated until the production converged.

### (3) Transition of Gramicidin S production in each cycle

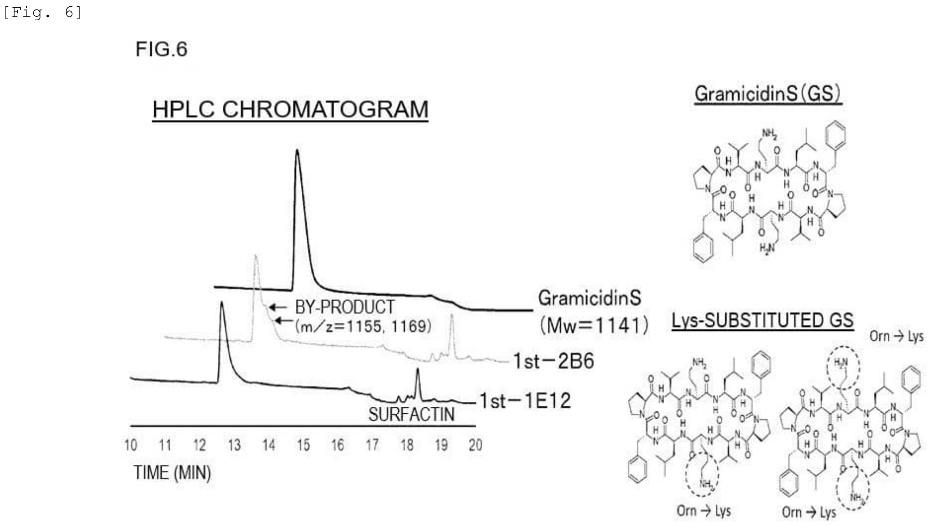

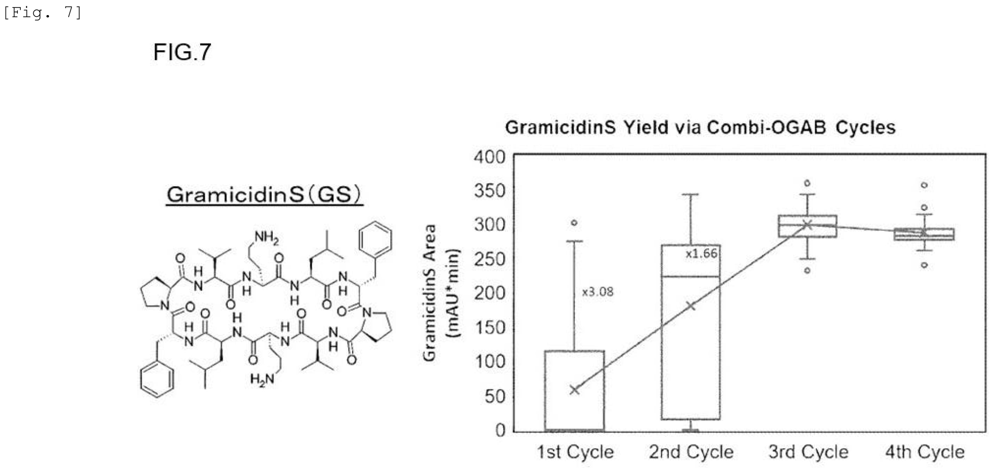

In the 1st cycle, when 192 strains were individually cultured, 134 strains grew. When extracts from those strains were analyzed by HPLC using the method described above, strains exhibiting single-peaked chromatograms and strains exhibiting triple-peaked chromatograms were observed (FIG. 6). When these peaks were analyzed by LC-MS, in addition to m/z 1141, which corresponds to the molecular weight of Gramicidin S as the target substance, m/z 1155 and 1169 were also detected from samples having triple-peaked chromatograms. It has been reported that the L-Orn-selective domain of the Gramicidin S biosynthetic enzyme group also has L-Lys selectivity (Non Patent Literature 13), and it has also been reported that, among wild-type production strains, there are strains that produce Lys1-substituted variants and Lys2-substituted variants (Non Patent Literature 14). Since only the promoter sequences were subjected to library construction in the present study, it was suggested that the transcription initiation timing or transcription strength of the promoters is effective in suppressing the production of Lys-substituted variants of Gramicidin S. To proceed to the 2nd cycle, re-culture of the strains that had highly produced Gramicidin S in the 1st cycle was attempted; however, most of the strains could not be re-cultured. Through this step, constructs with low reproducibility of culture due to incompatibility with the host could be easily eliminated. Plasmids were extracted from the seven strains that could be re-cultured, and the process proceeded to the 2nd cycle. In the 2nd cycle, 60 strains were cultured, and 59 strains grew. Among them, 45 strains were Gramicidin S-producing strains, and only two strains exhibited triple-peaked chromatograms on HPLC. From the 3rd cycle onward, all strains grew, and no Gramicidin S non-producing strains were observed, and in addition, no strains exhibiting triple-peaked chromatograms on HPLC were observed (Table 5). As a result of conducting the process through the 4th cycle, it was confirmed that the Gramicidin S-producing strains had converged by the 3rd cycle (FIG. 7). When the promoter sequences of the top five Gramicidin S-producing strains in the 3rd cycle were examined, all of the strains had the PsigW-grsT-Pcdd-grsA-PlytR-grsB promoter configuration. That is, the combination of PsigW (Class IV-weak), Pcdd (Class II-weak), and PlytR (Class III-strong) was demonstrated to be optimal for heterologous production of Gramicidin S by Bacillus subtilis.

**[Table 5]**

| | 1st cycle | 2nd cycle | 3rd cycle | 4th cycle |
|---|---|---|---|---|
| Number of seed plasmid strains | - | 7 | 7 | 5 |
| Number of cultured strains | 192 | 60 | 30 | 20 |
| Growth count (= HPLC analysis count) | 134 | 59 | 30 | 20 |
| Number of Gramicidin S-producing strains | 65 | 45 | 30 | 20 |

### (4) Analysis of purity of Gramicidin S produced by high-producing strains

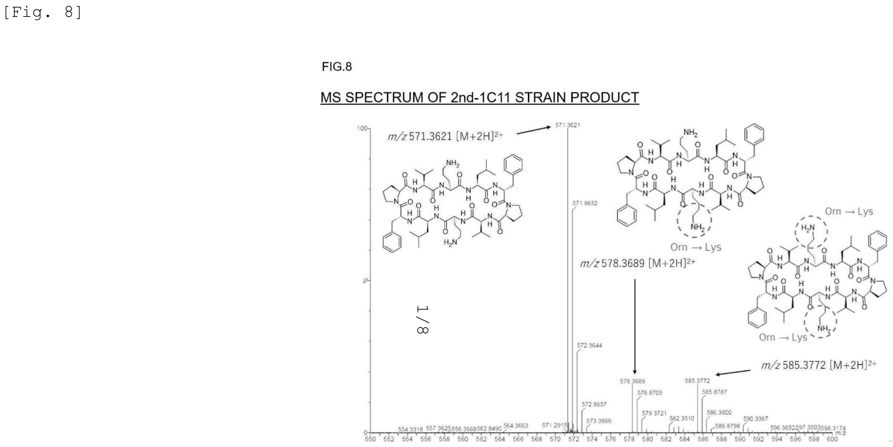

The biosynthetic extracts of strain C2 in the 3rd cycle (3rd-C2), which exhibited a single-peaked chromatogram on HPLC and was the highest-producing strain, and strain 1C11 in the 2nd cycle (2nd-1C11), which exhibited a triple-peaked chromatogram on HPLC, were analyzed by LC-MS and LC-MS/MS. In the 3rd-C2 strain, the ratio of the target Gramicidin S:Lys1-substituted variant:Lys2-substituted variant was 95.56:3.58:0.86, whereas in the 2nd-1C11 strain, the ratio was 75.63:14.62:9.75, confirming that the proportion of Lys-substituted variants as by-products was higher (FIG. 8, Table 6). That is, by optimizing the transcription initiation timing and transcription strength of the promoters for each gene by the Combi-OGAB^{™} method, it was possible not only to increase the production but also to search for promoter combinations that suppress the production of Lys-substituted variants. The target Gramicidin S and the Lys-substituted variants are detected at almost the same elution time on HPLC. Therefore, although considerable time and cost are required to improve the purity in the purification step, it was demonstrated that, through this step, promoter combinations capable of improving the purity of the target substance at the production stage, in addition to increasing the production, can be identified.

**[Table 6]**

| | 3rd-C2 | 2nd-1C11 |
|---|---|---|
| Promoter combination | PsigW-Pcdd-PlytR | PyqfD-PsigW-Pveg |
| GramicidinS (%) | 95.56% | 75.63% |
| Lys1-substituted variant (%) | 3.58% | 14.62% |
| Lys2-substituted variant (%) | 0.86% | 9.75% |

PKS and NRPS genes are present not only in Bacillus subtilis but also in large numbers in the genomes of actinomycetes and filamentous fungi. Accordingly, by conducting examinations as in the present Example, it is considered that similar experimental results can be obtained for the PKSs and NRPSs of such actinomycetes and filamentous fungi as well.

### (Example 3)

The present Example shows that promoter arrangement patterns (monocistronic and polycistronic), a single and a plurality of patterns of gene clusters, and a single pattern and a plurality of patterns of constructs are possible.

For the Gramicidin S biosynthetic gene cluster described in Example 2, three types of transcriptional arrangements were designed:
1) a construct in which a promoter is placed only upstream of grsT, and the entire cluster is transcribed polycistronically;
2) a construct in which promoters are placed upstream of grsT and upstream of grsA, grsT is transcribed monocistronically, and grsA and grsB are transcribed polycistronically; and
3) a construct in which promoters are placed upstream of grsT and upstream of grsB, grsT and grsA are transcribed polycistronically, and grsB is transcribed monocistronically, and
plasmids into which a plurality of types of growth phase-dependent promoters of Bacillus subtilis were introduced were prepared in the same manner as in Example 2.

In addition to the above three types, a plasmid library including four types of transcriptional arrangements was prepared by adding the plasmid containing the construct in which all of grsT, grsA, and grsB are transcribed monocistronically, as handled in Example 2, and by subjecting each plasmid, or mixtures thereof, to combinatorial library construction by the Combi-OGAB method. Through this process, screening of transcription units can be performed, in addition to screening of the transcription initiation timing and transcription strength of the promoters for each gene for high-level production of Gramicidin S.

### References

Reference Patent Literature 1: KR102301301B1
Reference Patent Literature 2: JP 2021-184730 A
Reference Patent Literature 3: JP 2019-198236 A
Reference Non Patent Literature 1: D. J. Newman, et. al., J. Nat. Prod., 83, 770 (2020)
Reference Non Patent Literature 2: C. C. Brett, et. al., Annu. Rev. Biochem., 90, 763 (2021)
Reference Non Patent Literature 3: Kang H-. S. et. al., ACS Synth. Biol., 5(9), 1002 (2016)
Reference Non Patent Literature 4: Kim H., et. al., ACS Synth. Biol., 9(1), 175 (2020)
Reference Non Patent Literature 5: Amagai K., et. al., Sci. Rep., 7, 3382 (2017)
Reference Non Patent Literature 6: Yang. S., et al., Appl. Microbiol. Biotechnol., 101 (10), 4151-4161 (2017)
Reference Non Patent Literature 7: Uozumi, T., et al. Molec. Gen. Genet., 152, 65-69 (1977)
Reference Non Patent Literature 8: Sambrook, J., et al., Molecular Cloning: A Laboratory Manual. Cold Spring Harbor Laboratory Press, Cold Spring Harbor, New York (1989)
Reference Non Patent Literature 9: Tsuge, K., et al., Nucleic Acids Res. 31, e133 (2003)
Reference Non Patent Literature 10: Grubbs, K. J., et al., mSystem s. 2(6) (2017)
Reference Non Patent Literature 11: Berditsch M., et al., App. Environ. Microbiol., 73(20), 6620 (2007)
Reference Non Patent Literature 12: Tsuge, K., et al., Arch Microbiol. 165, 243-251 (1996)
Reference Non Patent Literature 13: Stanisic. A., et al., Chem. Sci., 10, 10395-10399 (2019)
Reference Non Patent Literature 14: Alenezi F., Front. Microbiol., 8, 517-527, (2017)

### (Note)

As described above, although the present disclosure is exemplified by the use of preferred embodiments of the present disclosure, it is understood that the scope of the present disclosure should be interpreted solely based on the claims. It is also understood that any patent, any patent application, and any references cited herein should be incorporated herein by reference in the same manner as the contents are specifically described herein. The present application claims priority to Japanese Patent Application No. 2023-95819 filed to the Japan Patent Office, the entire contents of which are incorporated herein by reference.

### Industrial Applicability

The present disclosure can also provide a method for producing a microbial strain exhibiting a desired property such as high production and suppression of by-products by activating a desired gene cluster, such as genes constituting a natural product biosynthetic enzyme gene cluster, and through the method, can provide a substance that may be novel. These substances can be utilized in bio-industries such as pharmaceuticals, agriculture, fisheries, fragrances, foods, textiles, chemicals, and environmental applications.

### Sequence Listing Free Text

SEQ ID NO: 1: pGETS151 ΔBsmBI-pBR 
AAAAGGCCTTCTTGGCCGCCCTTCCCGGTCGATATGAACAGCTTATTTACATAA
TTCACGTTATTGGTAGTTATAAATGA
AATTCCTAATATCGGTTATGAAGTGAAATTGAATTTCTACTTGATCTTTCTCTCTATTTTT
GTAAAATAAAATTAAGAAT
ATTTAAATATTCAATGATTCATTTTTGCAGAAATCGGAGGAAGAATATATGAAAACAT
TTAACATTTCTCAACAAGA
TCCCCCCATATTGTTGTATAAGTGATGAAATACTGAATTTAAAACTTAGTTTATATGTGGT
AAAATGTTTTAATCAAGTT
TAGGAGGAATTAATTATGAAGTGTAATGAATAATGAATGTAACAGGGTTCAATTAAAAGAG
GGAAGCGTATCATTAACCC
TATAAACTACGTCTGCCCTCATTATTGGAGGGTGAAATGTGAATACATCCTATTCACAATC
GAATTTACGACACAACCAA
ATTTTAATTTGGCTTTGCATTTTATCTTTTTTTAGCGTATTAAATGAAATGGTTTTGAACG
TgTCATTACCTGATATTGC
AAATGATTTTAATAAACCACCAGCGAGTACAAACTGGGTGAACACAGCCTTTATGTTAACC
TTTTCCATTGGAACAGCTG
TATATGGAAAGCTATCTGATCAATTAGGCATCAAAAGGTTACTCCTATTTGGAATTATAAT
AAATTGTTTCGGGTCGGTA
ATTGGGTTTGTTGGCCATTCTTTCTTTTCCTTACTTATTATGGCTCGTTTTATTCAAGGGG
CTGGTGCAGCTGCATTTCC
AGCACTCGTAATGGTTGTAGTTGCGCGCTATATTCCAAAGGAAAATAGGGGTAAAGCATTT
GGTCTTATTGGATCGATAG
TAGCCATGGGAGAAGGAGTCGGTCCAGCGATTGGTGGAATGATAGCCCATTATATTCATTG
GTCCTATCTTCTACTCATT
CCTATGATAACAATTATCACTGTTCCGTTTCTTATGAAATTATTAAAGAAAGAAGTAAGGA
TAAAAGGTCATTTTGATAT
CAAAGGAATTATACTAATGTCTGTAGGCATTGTATTTTTTATGTTGTTTACAACATCATAT
AGCATTTCTTTTCTTATCG
TTAGCGTGCTGTCATTCCTGATATTTGTAAAACATATCAGGAAAGTAACAGATCCTTTTGT
TGATCCCGGATTAGGGAAA
AATATACCTTTTATGATTGGAGTTCTTTGTGGGGGAATTATATTTGGAACAGTAGCAGGGT
 
TTGTCTCTATGGTTCCTTA
TATGATGAAAGATGTTCACCAGCTAAGTACTGCCGAAATCGGAAGTGTAATTATTTTCCCT
GGAACAATGAGTGTCATTA
TTTTCGGCTACATTGGTGGGATACTTGTTGATAGAAGAGGTCCTTTATACGTGTTAAACAT
CGGAGTTACATTTCTTTCT
GTTAGCTTTTTAACTGCTTCCTTTCTTTTAGAAACAACATCATGGTTCATGACAATTATAA
TCGTATTTGTTTTAGGTGG
GCTTTCGTTCACCAAAACAGTTATATCAACAATTGTTTCAAGTAGCTTGAAACAGCAGGAA
GCTGGTGCTGGAATGAGTT
TGCTTAACTTTACCAGCTTTTTATCAGAGGGAACAGGTATTGCAATTGTAGGTGGTTTATT
ATCCATACCCTTACTTGAT
CAAAGGTTGTTACCTATGGAAGTTGATCAGTCAACTTATCTGTATAGTAATTTGTTATTAC
TTTTTTCAGGAATCATTGT
CATTAGTTGGCTGGTTACCTTGAATGTATATAAACATTCTCAAAGGGATTTCTAAATCGTT
AAGGGATCAACTTTGGGAG
AGAGTTCAAAATTGATCCTTTTTTTATAACAGGAATTGGGCATCAAATAAAACGAAAGGCT
CAGTCGAAAGACTGGGCCT
TTCGTTTTATCTGTTGTTTGTCGGTGAACGCTCTCCTGAGTAGGACAAATCCGCCGGGAGC
GGATTTGAACGTTGCGAAG
CAACGGCCCGGAGGGTGGCGGGCAGGACGCCCGCCATAAACTGCCAGGCATCAAATTAAGC
AGAAGGCCATCCTGACGGA
TGGCCTTTTTGCGTTTCTACAAACTCTTCCTGTCGTCATATCTACAATTCTACACAGCCCA
GTCCAGACTATTGAATTGT
ATCACGTTTTTGATATCCTACCAATAaCaAATTGATTGGAGGAATGCAAAGTGAATAATGA
ACCAGTAAAACGTGGTAAG
AAGAACAGATGGGAATTAAACCTACCTATAATGACTTATGTAGTAGCTGATGATTGGATTG
ATAAACTAGGACACGAAAC
GTTTACTTTATGGTTGAGGTTCCATACTTGGGTAGATAGAGAAGATGAACTCCGAGATTAT
GATCGCATACCTAGAAGTT
TTGAGAACATATATAAAAAGACACTAGGAATCTCAAAAAGTAAGTTTTATAGATTGATAAA
ACCTTTATGGGAATATGGA
TTAATAGACATCATAGAATACGAAGAATCTAACCGTAATTCTACTAAACCTAAAAATATAA
TTGTTTATGAGTATCCTTT
ACACGAAATAGAAAGAAAGTATAAACCACTAGAAAAATTAAGAGATTGGGATAAAGACTAT
AATTCCGTTTCTAAAGAAT
TAGGTAAAACAGGTGGTAGACCAAGGAAAAAAGATAGTGAAGAAGAACCCGAAAAGAAACC
CGAAGAAGTAACTAAAAAG
AAACGTAAATATAAGTTAAAAAGAGTTATCCACAACGGTTTCAAAAATGAAACGGTGGAGG
GTTTCAAAAATGAAACGGT
 
GGAGGGTTTCAAAAATGAAACGGTGACCGTTTCAAAAATAAAACCCAATAATTATTCAAAT
ATCTTTAATAACTTATCAA
ATATTTCTACTAATGTTTCAAATAATTTATTAATTGATGATGATGAGGAAATCGAAAATGA
ACCAACTGGTCGTACAATA
AATAGGTCATTACTTTTTTCACAAGAAGATATTAAACAGGCCTATCAATTTATTAATAGAT
TTTCAGTTATACAGTTACG
TGAAAACTTTAGCTTTGATAAACACTTTGAAGAACGGTTGGTATGTTATTTATGGAAAGCA
GGGATTTCTACTTTTTACA
CGCACGAAATCAGTAAAATGATAAAAAAAATAGCAGACTATGAAAAATCTAAAAAAGGTAG
ATTAAACCCAATACGTGAC
CGAGCCTTATATATGGTAAATGGTCTTGTAATGAATAGAGCTTCTTCCCAAAGTGAACATG
CTACTTATAAACTAAACCA
ATATAAAAAACAGAAGGAACAGGAAAAACAACAACAGGAGCAACAAAGATCAAGAGTACCG
TTCTATAATTGGTTGGAGG
AAAGAGAAGAACAAACCGAAGGTCAACTACCCACCACTTAAGCGGCCGCAAGCTTGAAGAG
CTCTTCTTTCAGAACGCTC
GGTTGCCGCCGGGCGTTTTTTATGAGAtGTCTCGGCCTGTTTGGCCATTAacgtgcaggtg
gatccagatctaagcttct
atagaagcttggtaccgacgtctcggcctgtttggcccgccgcatccataccgccagttgt
ttaccctcacaacgttcca
gtaaccgggcatgttcatcatcagtaacccgtatcgtgagcatcctctctcgtttcatcgg
tatcattacccccatgaac
agaaatcccccttacacggaggcatcagtgaccaaacaggaaaaaaccgcccttaacatgg
cccgctttatcagaagcca
gacattaacgcttctggagaaactcaacgagctggacgcggatgaacaggcagacatctgt
gaatcgcttcacgaccacg
ctgatgagctttaccgcagctgcctcgcgcgtttcggtgatgacggtgaaaacctctgaca
catgcagctcccgaagacg
gtcacagcttgtctgtaagcggatgccgggagcagacaagcccgtcagggcgcgtcagcgg
gtgttggcgggtgtcgggg
cgcagccatgacccagtcacgtagcgatagcggagtgtatactggcttaactatgcggcat
cagagcagattgtactgag
agtgcaccatatgcggtgtgaaataccgcacagatgcgtaaggagaaaataccgcatcagg
cactcttccgcttcctcgc
tcactgactcgctgcgctcggtcgttcggctgcggcgagcggtatcagctcactcaaaggc
ggtaatacggttatccaca
gaatcaggggataacgcaggaaagaacatgtgagcaaaaggccagcaaaaggccaggaacc
gtaaaaaggccgcgttgct
ggcgtttttccataggctccgcccccctgacgagcatcacaaaaatcgacgctcaagtcag
 
aggtggcgaaacccgacag
gactataaagataccaggcgtttccccctggaagctccctcgtgcgctctcctgttccgac
cctgccgcttaccggatac
ctgtccgcctttctcccttcgggaagcgtggcgctttctcatagctcacgctgtaggtatc
tcagttcggtgtaggtcgt
tcgctccaagctgggctgtgtgcacgaaccccccgttcagcccgaccgctgcgccttatcc
ggtaactatcgtcttgagt
ccaacccggtaagacacgacttatcgccactggcagcagccactggtaacaggattagcag
agcgaggtatgtaggcggt
gctacagagttcttgaagtggtggcctaactacggctacactagaaggacagtatttggta
tctgcgctctgctgaagcc
agttaccttcggaaaaagagttggtagctcttgatccggcaaacaaaccaccgctggtagc
ggtggtttttttgtttgca
agcagcagattacgcgcagaaaaaaaggatctcaagaagatcctttgatcttttctacggg
gtctgacgctcagtggaac
gaaaactcacgttaagggattttggtcatgagattatcaaaaaggatcttcacctagatcc
ttttaaattaaaaatgaag
ttttaaatcaatctaaagtatatatgagtaaacttggtctgacagttaccaatgcttaatc
agtgaggcacctatctcag
cgatctgtctatttcgttcatccatagttgcctgactccccgtcgtgtagataactacgat
acgggagggcttaccatct
ggccccagtgctgcaatgataccgcgagacccacgctcaccggctccagatttatcagcaa
taaaccagccagccggaag
ggccgagcgcagaagtggtcctgcaactttatccgcctccatccagtctattaattgttgc
cgggaagctagagtaagta
gttcgccagttaatagtttgcgcaacgttgttgccattgctgcaggcatcgtggtgtcacg
ctcgtcgtttggtatggct
tcattcagctccggttcccaacgatcaaggcgagttacatgatcccccatgttgtgcaaaa
aagcggttagctccttcgg
tcctccgatcgttgtcagaagtaagttggccgcagtgttatcactcatggttatggcagca
ctgcataattctcttactg
tcatgccatccgtaagatgcttttctgtgactggtgagtactcaaccaagtcattctgaga
atagtgtatgcggcgaccg
agttgctcttgcccggcgtcaacacgggataataccgcgccacatagcagaactttaaaag
tgctcatcattggaaaacg
ttcttcggggcgaaaactctcaaggatcttaccgctgttgagatccagttcgatgtaaccc
actcgtgcacccaactgat
cttcagcatcttttactttcaccagcgtttctgggtgagcaaaaacaggaaggcaaaatgc
cgcaaaaaagggaataagg
 
gcgacacggaaatgttgaatactcatactcttcctttttcaatattattgaagcatttatc
agggttattgtctcatgag
cggatacatatttgaatgtatttagaaaaataaacaaataggggttccgcgcacatttccc
cgaaaagtgccacctggat ccacctgcacgt
SEQ ID NO: 2: Triketide Pyorne BGC 
ATCGATCGGCAGGTGATGTCAATAACACCTGCATCGGGAATATGCAAAGAGGTG
ATCGCATGGCGTTTATTTTATCCATT
GGAACAAGTCTaCCTGCGTATAATGTAAACCAAGAGAAAGCGGCCGAGTTCGCCCGCTATA
TGTTTCAGCATTCTTTTAA
GGATATTGACAGGCTGTTATCCTCCTTTAAAAACGGGCAAATCCATTCGAGACAGTTTGTT
AAGCCGATTGAATGGTATA
AAGAGGGGCACAGCTTTGAAGAAAAAAATCAAATATACATAGAAGAAACACTTAAACACAG
CAGAGCGGCTGTTCGTGAG
TGCCTCTCTCATCCGGAATTTTTTCAAGAAGCCATTCCTTATGAGAAAGTGGAAGCTGTTT
TTTTCGTgTCCAGTACAGG
TCTTTCCACACCGAGTATCGAGGCGCGGCTTATGAATGAACTTCCGTTTTCTCCATATACG
AAGCGGATTCCGATTTGGG
GGCTTGGCTGTGCAGGAGGAGCCAGCGGACTTGCGCGTGCAGCCGAATACTGCAAAGCGTA
TCCTGAGGCTTTTGTGCTT
GTCATTTCTGCCGAGCTGTGCAGCCTGACATTCCAGCCGGAAGATAAAACAAAAAGCAATC
TGATCGGCACCTCGCTTTT
TGGTGACGGCATTGCCGCAGCTTTATTATGCGGAGAGAAGGCGGACCGCAGGGTGTCAAAG
CTGAAGCTTGCGCCGAAGA
TAATGGACGCCCAATCTGTTTTGATGAAACAATCAGAAGATGTCATGGGCTGGGACTTTAC
CGATCAAGGATTCAAAGTC
ATTTTCTCACGAGACATTCCGACCTTGGTGGAAAAATGGCTCAAAACAAATGTACAGATTT
TTTTGGACAAACATAAGCT
GTCATTCCATGATATCAGTGTTTTTTTGGCGCATCCAGGCGGAAAAAAAGTGATCGATGCA
TACATCAAAAGTCTCGGGC
TGTCGTCAGAAAAGCTTTCATCTGCGCAAAGCATTTTGCAAAAGCACGGAAATATGTCaTC
AGCCACAATCTTGTATGTG
ATCAAAGACCATCTGCAGAACGGACATAAAAAGGAAGCTGAACGAGGACTGATTGGCGCAT
TAGGGCCGGGTTTCTCATC
AGAGTTGCTATTATTCAGCTGGGAAAAGGGGGCCTGAGGCCTTGATGGCCATCGAGAGACC
AATAATAATAGGTCTCAGG
AAAAGGGGGCCTGACTCATGTTTTGGTTGTTGATCGCCATTCTCATTGTTCAAAGAGCAGC
AGAAATGGCAGTGGCCAGG
CAAAACGAACAAAAGGTAAAGAAGCAAGGAGCAATTGAATTTGGCGAGAGCCACTATCCGT
ATATCATTACGATGCATAT
 
CCTTTTTTTCCTGTCCTTAATTGCCGAGGTGCTGCTGATGAATAAACAGCCGTCCAGCTGG
TGGCTCGGTATCGCTGCAG
TGATTTTAAGCGTACAGATCGTACGGTATTGGGCGCTTTGTTCCTTAGGAGCATATTGGAA
TACGAAAATCCTTGTTGTT
CCAGGTGTAGAGCTTGTAAAAAAAGGTCCATATAAATGGATGAAACATCCAAACTATGCCG
TTGTTATCCTCGAAATTTT
GCTGATTCCGCTGCTTTATCAAGCATACGTCACCATGTGTCTGTTTTCCATTTTCAATGCC
GTGTTGTTAAGCGTCAGAA
TTCGCGCAGAgGACAAAGCATTACAGGAATACAGCGTAAAATAGaagaagaagtgtgaaaa
agcgcagctgaaatagctg cgcttttttgtgtcataa
SEQ ID NO: 3: GramicidinS BGC 
ATCGATCGGCAGGTGATGTCAATAACACCTGCATCGGGAAGAATAAAGGGAGAG
GTGAATGTGACTTTTATTTCACAAGT
AAATAAATGGTTTGTTAATGCTAATGTTAACTCAGCTGCAAAGCTTAGGCTATTCTGTATT
CCATATGCAGGCGGTGGTG
CTTCCGCCTTTTATGAATGGAGTCATTTTTTTCCAAAGGAAATTGAAGTTTGTTCAATTCA
ATTACCTGGAAGGGAAAAT
AGGGGGGCGGAAGTTCCGCTAACAAATTTACAACAGATAGTAGAAATAGTAGCTGAGGAAA
TACAACCATTAATAAATAT
TCCATTTGCTTTTTTGGGGCATAGCATGGGAGCATTAATAAGTTTTGAACTGGCTCGCACA
ATACGGCAAAAGAGTAATG
TTAATCCGGTTCACTTGTTTGTTTCAGGGCGACATGCACCTCAAATCCCATGTGCAAAACA
AGACTATCATTTACTTCCC
GATGAACAATTTATACAAGAATTGCGTTCATTGAATGGAACTCCAGAGATAGTATTACAAG
ACGCAGAGATGATGAGTAT
ATTACTCCCAAGACTTCGGGCTGATTTTTCTGTGTGTGGCTCCTATCAGTACAAAAACGAC
GAGCCTTTTGAATGCCCAA
TCACTGCTTTTGGAGGAAAAAATGATAATGGTGTTACTTATCAATCATTAGAAGCCTGGAG
AGAGCAAACCAAGAGGGAA
TTTTCTGTGTGTATGTATCCAGGTGATCATTTTTTTCTTTACGAAAGCAAATATGAAATGA
TTGAGTTCATGTGTAAACA
ATTACGTTTAGTATTAGCTCCTAAAATATAAggcctTGAtggccATCGAGAGACCAATAAT
AATAGGTCTCAGGAACTAT
TTTTTACAGGGGGTATATatgTTAAACAGTTCTAAAAGTATATTGATTCATGCTCAAAATA
AAAATGGAACGCATGAAGA
GGAGCAGTATCTCTTTGCTGTGAACAACACCAAAGCGGAGTATCCACGTGATAAGACGATC
CATCAGTTATTTGAgGAGC
AGGTTAGTAAGAGgCCAAACAATGTAGCCATTGTATGTGAAAATGAGCAACTTACCTACCA
TGAGCTTAATGTGAAAGCC
 
AATCAACTAGCACGGATTTTTATAGAAAAAGGGATTGGAAAAGACACTCTTGTTGGAATTA
TGATGGAGAAATCTATCGA
TTTATTTATAGGCATATTAGCCGTTTTAAAAGCtGGTGGAGCATATGTTCCGATTGATATT
GAATATCCTAAGGAAAGAA
TTCAATATATTCTTGATGATAGTCAGGCAAGAATGCTACTTACCCAGAAGCATTTGGTTCA
TTTAATTCATAATATTCAA
TTTAATGGGCAAGTGGAAATTTTTGAAGAAGATACTATCAAAATTAGAGAAGGAACTAATC
TACATGTACCAAGTAAATC
AACCGATCTTGCTTATGTTATTTATACTTCTGGTACAACAGGCAATCCAAAAGGTACAATG
CTGGAGCATAAAGGAATAA
GTAATCTAAAGGTATTTTTCGAAAATAGTCTTAACGTGACTGAAAAGGATAGAATTGGTCA
ATTTGCCAGCATCTCTTTT
GATGCATCTGTATGGGAGATGTTTATGGCTTTGTTAACGGGGGCTAGCCTGTATATTATCC
TGAAGGATACAATCAATGA
TTTTGTGAAGTTTGAACAATACATTAACCAAAAGGAAATCACTGTTATTACGTTACCACCT
ACCTATGTAGTTCATCTTG
ATCCAGAACGTATTTTATCGATACAAACGTTAATTACAGCAGGCTCAGCTACCTCGCCTTC
CTTAGTAAACAAGTGGAAG
GAGAAAGTAACTTACATAAATGCCTATGGCCCTACGGAAACAACTATTTGTGCGACTACAT
GGGTAGCCACCAAAGAAAC
AATAGGTCATTCAGTTCCAATCGGAGCACCAATTCAAAATACACAAATTTATATTGTCGAT
GAAAATCTTCAATTAAAAT
CGGTTGGTGAAGCTGGTGAATTGTGTATTGGTGGAGAAGGGTTAGCAAGGGGATATTGGAA
GCGACCGGAATTAACTTCC
CAGAAGTTCGTTGATAACCCGTTTGTTCCAGGAGAGAAGTTGTATAAAACAGGAGATCAGG
CAAGATGGCTATCTGATGG
AAATATTGAATATCTCGGAAGAATAGATAACCAGGTAAAGATTAGAGGTCACCGAGTTGAA
CTAGAAGAAGTTGAGTCTA
TTCTTCTAAAGCATATGTATATTAGCGAAACTGCAGTAAGTGTGCATAAAGATCACCAAGA
ACAGCCGTATTTGTGCGCT
TATTTTGTATCGGAAAAGCATATACCACTAGAACAGTTAAGACAATTCTCATCAGAAGAAC
TGCCAACGTATATGATCCC
TTCTTATTTTATCCAGTTAGACAAAATGCCGCTTACATCAAATGGGAAGATTGATCGAAAG
CAGTTGCCGGAACCTGATT
TAACTTTCGGGATGAGGGTAGACTATGAAGCGCCGCGAAATGAAATCGAGGAAACGCTTGT
TACTATCTGGCAGGATGTA
TTAGGTATTGAGAAAATCGGTATTAAAGATAATTTCTATGCATTAGGTGGAGATTCTATTA
AAGCAATACAGGTTGCTGC
TCGCCTGCATTCCTACCAATTAAAGCTAGAAACAAAAGATTTATTAAAGTATCCAACAATC
 
GATCAACTCGTTCATTATA
TAAAAGATAGTAAAAGAAGAAGTGAGCAAGGTATTGTGGAAGGTGAGATTGGACTTACACC
TATTCAGCATTGGTTCTTT
GAACAACAATTTACAAATATGCACCATTGGAACCAATCGTATATGTTGTATAGACCAAATG
GGTTTGATAAAGAGATCTT
GCTAAGGGTATTTAATAAAATTGTTGAGCATCATGATGCATTACGTATGATATACAAACAT
CATAACGGAAAGATCGTGC
AGATAAATCGGGGGCTTGAAGGTACGTTGTTTGATTTTTATACCTTTGATTTAACTGCAAA
TGATAATGAGCAACAGGTG
ATTTGTGAAGAATCTGCTCGATTACAAAATAGTATAAACTTGGAAGTAGGCCCTCTAGTAA
AGATAGCGCTGTTTCATAC
TCAGAATGGAGATCACCTGTTTATGGCTATTCATCATTTGGTTGTGGATGGTATTTCTTGG
AGGATTTTGTTTGAGGATT
TGGCCACAGCTTATGAACAAGCAATGCATCAGCAAACGATTGCTTTACCAGAGAAAACAGA
TTCATTTAAGGACTGGTCT
ATTGAATTAGAAAAATATGCGAACAGCGAATTATTCCTAGAAGAAGCTGAATATTGGCATC
ATTTGAATTATTATACCGA
GAACGTTCAAATTAAGAAAGATTATGTCACCATGAACAATAAACAAAAGAATATACGTTAT
GTAGGAATGGAGTTAACAA
TAGAAGAGACAGAAAAATTATTGAAAAATGTAAATAAAGCGTATCGAACAGAAATTAATGA
TATTTTATTAACGGCACTT
GGCTTTGCACTCAAAGAATGGGCCGATATTGATAAAATTGTAATTAACTTAGAGGGACACG
GACGGGAAGAAATACTGGA
ACAGATGAACATTGCAAGGACGGTAGGCTGGTTTACTTCCCAGTATCCTGTTGTACTTGAT
ATGCAAAAATCGGATGATT
TGTCTTATCAAATCAAATTAATGAAAGAAAATTTACGCAGAATACCTAACAAAGGAATCGG
ATATGAAATTTTTAAGTAT
TTAACAACTGAATATTTACGGCCTGTTTTACCCTTTACATTAAAGCCGGAAATTAACTTTA
ACTACTTAGGACAGTTCGA
TACGGACGTGAAaACTGAATTGTTTACTCGTTCTCCTTATAGCATGGGTAATTCATTAGGA
CCAGATGGAAAAAATAATT
TAAGCCCAGAAGGGGAAAGTTATTTTGTACTCAATATTAATGGTTTTATTGAAGAAGGTAA
GCTTCACATCACCTTTTCT
TATAATGAACAGCAGTATAAGGAGGATACCATTCAGCAATTGAGCCGGAGCTATAAGCAAC
ATCTTTTGGCCATCATTGA
ACATTGTGTACAGAAGGAAGATACTGAGTTAACTCCAAGTGATTTCAGTTTCAAGGAACTT
GAATTAGAAGAGATGGATG
ATATTTTCGATTTGTTGGCCGATTCATTAACGtaaggcctCGAtggccATCGATGTCTTCT
TATTATTATGAAGACATGG
 
AACATTATTATGAGGTGCTAGCATGAGTACATTTAAAAAAGAACATGTTCAGGATATGTAT
CGTTTATCTCCCATGCAGG
AAGGCATGTTGTTTCACGCATTACTTGATAAAGATAAAAATGCTCACCTGGTACAAATGTC
TATCGCGATCGAAGGTATC
GTGGATGTGGAGCTGCTTAGTGAAAGCTTGAACATATTGATTGATAGATACGATGTGTTTA
GAACAACATTCTTACATGA
AAAAATTAAACAACCGCTTCAGGTAGTGCTAAAGGAACGGCCTGTTCAGCTTCAATTTAAA
GACATATCATCCTTAGATG
AAGAAAAAAGAGAACAGGCTATTGAGCAGTATAAGTATCAAGATGGGGAAACAGTCTTTGA
TTTAACAAGAGATCCCTTG
ATGAGAGTAGCTATTTTTCAAACTGGTAAGGTTAACTACCAAATGATCTGGAGCTTCCACC
ATATTTTAATGGATGGTTG
GTGCTTCAACATTATATTTAATGACTTGTTCAATATATATCTGTCATTAAAAGAGAAGAAA
CCTCTTCAGTTAGAGGCGG
TGCAACCATATAAGCAGTTTATTAAGTGGCTTGAAAAACAAGATAAACAGGAAGCaCTTCG
CTACTGGAAAGAACATTTA
ATGAATTATGATCAATCAGTAACATTACCTAAAAAGAAAGCAGCTATTAATAATACTACAT
ATGAACCAGCACAGTTTCG
TTTTGCGTTTGACAAAGTGCTTACCCAGCAGCTGCTTCGTATTGCCAATCAAAGCCAAGTA
ACACTAAATATTGTTTTTC
AAACAATATGGGGGATTGTACTTCAAAAATACAATTCCACTAATGATGTTGTATATGGCTC
TGTTGTATCAGGCCGTCCT
TCTGAAATATCGGGAATTGAGAAAATGGTTGGACTATTTATTAATACTCTTCCATTACGTA
TCCAAACGCAAAAAGATCA
ATCATTTATTGAATTAGTAAAGACTGTTCATCAAAACGTCCTTTTCTCGCAACAGCATGAG
TATTTTCCATTGTATGAAA
TACAAAATCATACAGAATTAAAACAGAATCTGATTGATCATATTATGGTAATTGAAAATTA
TCCTTTAGTAGAAGAATTG
CAAAAGAATAGTATCATGCAAAAAGTAGGGTTTACAGTTCGTGATGTCAAAATGTTTGAAC
CAACTAATTATGATATGAC
AGTTATGGTTTTACCTCGTGATGAAATTAGTGTCCGACTCGATTATAACGCAGCCGTTTAT
GATATAGATTTCATAAAAA
AAATTGAAGGTCACATGAAAGAAGTGGCTTTATGCGTGGCAAATAATCCACATGTGTTAGT
ACAGGACGTTCCTCTGCTT
ACAAAGCAAGAAAAACAACATTTATTGGTAGAGCTGCATGATTCGATAACAGAGTATCCTG
ATAAGACGATTCATCAGTT
ATTTACAGAACAGGTAGAAAAAACACCAGAGCATGTGGCAGTTGTATTCGAAGATGAGAAA
GTGACCTATAGAGAGCTGC
ATGAGAGATCTAATCAATTAGCCAGATTCTTAAGAGAAAAAGGCGTAAAAAAAGAAAGCAT
 
CATAGGCATTATGATGGAG
CGTTCAGTTGAAATGATTGTTGGGATCTTAGGGATTTTAAAAGCTGGTGGAGCTTTTGTGC
CTATTGATCCTGAATATCC
AAAAGAAAGAATCGGCTATATGTTAGATTCTGTACGGCTAGTACTTACACAACGCCATTTA
AAGGATAAATTTGCTTTTA
CGAAAGAAACGATAGTAATTGAAGATCCAAGTATTTCACACGAGTTAACTGAAGAAATAGA
TTATATTAATGAATCAGAG
GACTTGTTTTATATTATTTATACATCAGGAACAACAGGTAAACCAAAAGGGGTTATGCTAG
AGCACAAAAACATCGTTAA
TCTGCTTCATTTTACTTTCGAGAAAACAAATATCAACTTTAGTGACAAAGTATTACAGTAT
ACAACATGCAGTTTTGACG
TGTGTTACCAAGAAATTTTTTCGACGCTCTTGTCTGGAGGGCAATTATATCTTATTAGGAA
AGAAACTCAACGCGATGTA
GAGCAATTATTTGATTTAGTAAAACGTGAAAATATTGAAGTATTATCCTTTCCTGTGGCTT
TTCTAAAATTTATTTTCAA
TGAAAGAGAATTTATCAATCGTTTTCCAACTTGCGTGAAACATATTATCACAGCAGGAGAA
CAATTAGTAGTTAACAATG
AGTTTAAACGTTATTTGCATGAACATAACGTACATTTACACAATCATTATGGTCCATCAGA
AACGCATGTTGTTACCACC
TATACTATTAATCCTGAAGCTGAAATTCCTGAATTACCACCGATAGGAAAACCTATCTCCA
ATACATGGATTTATATTTT
GGATCAAGAACAACAACTACAACCACAAGGAATTGTAGGAGAGTTATATATTTCGGGCGCA
AATGTTGGAAGAGGATATT
TGAATAATCAAGAATTAACGGCAGAAAAATTCTTTGCAGATCCCTTTAGGCCAAACGAACG
GATGTACCGAACAGGGGAT
TTAGCAAGGTGGTTGCCAGACGGAAATATCGAATTTTTAGGAAGgGCCGATCATCAGGTGA
AAATTAGGGGGCATCGAAT
AGAGCTTGGTGAGATCGAGGCACAATTATTAAATTGTAAGGGTGTAAAAGAAGCTGTTGTT
ATCGATAAAGCGGATGATA
AAGGCGGAAAATATTTATGTGCCTATGTTGTTATGGAAGTAGAAGTAAATGACTCTGAGCT
TCGAGAATATTTGGGGAAA
GCTTTGCCTGATTATATGATCCCGTCGTTCTTTGTTCCGTTGGATCAGCTGCCGCTTACAC
CAAACGGAAAAATAGACAG
AAAATCTCTTCCGAATCTAGAGGGGATTGTGAATACAAACGCAAAATATGTAGTACCTACA
AATGAGCTGGAAGAAAAAT
TGGCTAAAATCTGGGAAGAAGTACTTGGGATTTCTCAGATCGGTATACAAGACAATTTCTT
TTCGTTAGGCGGGCATTCT
CTTAAAGCCATTACGCTTATTTCCCGTATGAACAAAGAGTGTAATGTAGACATTCCTCTAC
GTTTGTTATTTGAAGCACC
 
AACCATTCAGGAAATCTCTAATTATATAAACGGGGCAAAGAAAGAAAGCTATGTTGCCATT
CAGCCTGTACCAGAACAAG
AGTACTATCCTGTATCATCAGTTCAAAAAAGAATGTTTATTCTTAATGAATTTGATCGTTC
AGGTACGGCCTATAATTTA
CCTGGTGTTATGTTTCTAGATGGAAAATTGAACTACCGACAATTGGAAGCAGCGGTAAAAA
AATTAGTTGAGCGACATGA
AGCGCTGCGTACTTCCTTTCATTCAATTAATGGGGAACCAGTTCAGCGGGTGCATCAAAAT
GTAGAACTGCAGATTGCTT
ATTCAGAGTCAACGGAAGATCAGGTGGAGCGAATTATTGCGGAATTTATGCAACCATTTGC
TCTTGAAGTTGCTCCGTTA
CTTCGTGTAGGTCTTGTTAAATTGGAGGCAGAACGTCATCTATTTATAATGGATATGCATC
ATATCATCTCGGATGGGGT
ATCCATGCAGATCATGATTCAAGAAATTGCTGATTTGTATAAAGAAAAGGAACTTCCTACG
TTAGGCATTCAATATAAAG
ACTTTACTGTTTGGCATAATCGCTTGCTTCAATCGGATGTTATTGAAAAACAAGAAGCTTA
CTGGCTGAACGTATTTGCA
GAAGAGATTCCAGTATTGAATCTACCGACCGATTACCCAAGACCAACCATTCAAAGCTTTG
ATGGTAAAAGATTTACATT
CAGTACAGGAAAGCAGCTTATGGATGATTTATACAAGGTGGCAACAGAAACAGGAACAACA
CTATATATGGTTTTACTTG
CTGCGTATAATGTTTTCTTATCGAAGTATTCCGGGCAAGATGACATCGTTGTAGGAACACC
GATTGCTGGTAGGTCcCAT
GCTGATGTGGAAAATATGCTGGGGATGTTTGTAAATACATTAGCAATAAGAAGTCGTTTAA
ATAATGAGGATACTTTTAA
AGATTTTTTAGCAAATGTAAAACAAACGGCTTTGCATGCCTATGAAAATCCAGATTACCCA
TTTGATACGCTTGTCGAAA
AGTTGGGTATACAGAGAGATTTAAGTAGAAATCCATTATTTGATACGATGTTTGTTTTGCA
AAATACGGATAGAAAGTCT
TTTGAGGTTGAACAGATAACGATTACACCATATGTTCCAAATAGCAGACATTCTAAATTTG
ATCTTACATTAGAGGTTAG
CGAAGAACAAAATGAGATTTTATTATGCCTAGAATATTGCACTAAATTATTTACGGATAAA
ACAGTTGAAAGAATGGCTG
GTCATTTTTTACAGATCTTGCATGCAATTGTTGGGAACCCAACGATTATAATATCAGAAAT
CGAGATATTGTCTGAAGAA
GAAAAACAACATATTTTATTCGAGTTCAACGATACGAAAACCACATATCCACATATGCAAA
CAATTCAAGGATTATTTGA
GGAACAGGTGGAGAAaACGCCCGACCATGTTGCAGTTGGATGGAAAGACCAAACATTAACG
TATCGGGAACTTAACGAAA
GAGCGAATCAGGTCGCAAGAGTCTTACGGCAAAAAGGAGTCCAACCCGATAATATCGTGGG
 
ATTGCTGGTTGAGCGTTCA
CCTGAAATGCTCGTGGGTATCATGGGAATTCTTAAAGCAGGGGGAGCTTATTTACCTCTTG
ATCCGGAGTACCCAGCGGA
TAGAATTTCGTACATGATACAAGATTGTGGTGTACGCATTATGCTTACCCAACAGCATCTT
TTATCTTTAGTACATGATG
AATTTGATTGTGTTATTTTGGATGAgGACAGTTTGTACAAGGGGGATTCTTCCAATTTGGC
TCCGGTTAACCAGGCCGGG
GATTTAGCCTACATCATGTACACTTCTGGTTCTACAGGAAAGCCTAAAGGTGTTATGGTAG
AACATCGAAATGTGATTCG
CCTTGTGAAAAATACAAATTATGTTCAGGTCCGCGAgGACGATCGTATAATACAGACCGGA
GCAATTGGATTCGATGCAC
TGACATTTGAAGTTTTTGGCTCATTGCTGCATGGAGCTGAATTGTATCCTGTTACTAAAGA
CGTGCTATTAGATGCAGAG
AAACTACACAAATTTTTACAAGCGAATCAAATTACGATTATGTGGTTAACTTCTCCGTTAT
TTAACCAATTGTCACAAGG
AACCGAAGAGATGTTTGCTGGCCTTCGCTCCCTAATTGTAGGTGGAGATGCCTTGTCTCCG
AAACACATCAATAATGTAA
AGCGAAAATGCCCTAATCTGACTATGTGGAACGGTTACGGCCCAACAGAAAACACCACTTT
TTCTACATGCTTTCTTATT
GATAAAGAATATGATGACAATATTCCGATAGGGAAGGCCATTAGTAATTCAACAGTGTATA
TCATGGACCGGTATGGCCA
GCTTCAGCCGGTGGGTGTACCAGGAGAATTATGTGTAGGAGGGGATGGGGTTGCCAGGGGA
TATATGAATCAGCCTGCAT
TAACAGAAGAGAAGTTTGTCCCAAATCCATTCGCTCCTGGTGAGAGAATGTATCGCACGGG
GGATTTGGCAAGATGGTTG
CCTGATGGAACAATTGAGTATTTAGGTCGTATTGATCAGCAaGTGAAAATCAGGGGCTACC
GTATTGAACCGGGAGAGAT
TGAAACGCTTCTTGTGAAGCACAAAAAAGTCAAAGAATCGGTAATCATGGTAGTAGAGGAT
AATAATGGACAAAAGGCTC TATG
        CGCTTATTACGTTCCGGAAGAAGAAGTAACGGTATCTGAACTGAGGGAATATAT
AGCTAAAGAGTTGCCTGTTTAC
ATGGTTCCAGCCTATTTTGTACAGATTGAACAAATGCCTCTTACACAGAACGGTAAAGTAA
ATCGAAGCGCGTTACCAAA A
        CCAGATGGTGAATTTGGTACAGCAACCGAATATGTAGCGCCTAGCAGCGACATT
GAAATGAAGCTGGCAGAGATTTGGCA
TAATGTGTTAGGGGTAAACAAAATCGGGGTACTGGATAACTTCTTTGAATTAGGTGGTCAT
TCATTAAGAGCTATGACAA
TGATTTCCCAGGTACATAAAGAGTTCGACGTTGAATTGCCATTAAAAGTGTTATTTGAAAC
ACCAACGATCTCTGCATTA
 
GCTCAATACATTGCTGATGGAGAAAAAGGAATGTACCTGGCCATTCAACCTGTTACCCCGC
AGGATTACTATCCAGTATC
ATCTGCGCAAAAGAGGATGTACATCCTTTATGAATTTGAAGGGGCTGGCATTACCTATAAT
GTACCTAATGTAATGTTTA
TAGAAGGAAAGCTGGATTATCAGCGCTTTGAATACGCTATAAAAAGTTTGGTAAATCGACA
TGAGGCGCTTCGAACGTCT
TTCTATTCGCTTAATGGAGAACCAGTTCAGCGTGTACATCAAAATGTAGAGCTACAGATTG
CTTATTCGGAGGCGAAAGA
AGATGAGATAGAGCAAATTGTAGAAAGCTTTGTTCAACCATTTGACCTTGAAATAGCTCCG
CTGCTTCGCGTAGGGCTTG
TTAAATTGGCATCGGATCGCTATTTATTCCTAATGGATATGCATCATATTATCTCAGATGG
TGTATCAATGCAAATTATA
ACAAAAGAAATTGCCGACTTATATAAAGGAAAAGAGCTTGCTGAACTGCATATTCAGTATA
AAGATTTTGCTGTATGGCA
AAACGAATGGTTTCAATCTGACGCTCTTGAAAAACAGAAAACGTATTGGTTGAACACCTTT
GCAGAGGATATTCCGGTTT
TAAATTTGTCAACTGATTATCCAAGACCGACAATTCAAAGTTTTGAAGGAGATATTGTCAC
GTTTAGTGCAGGGAAGCAA
CTTGCGGAAGAATTGAAACGCCTGGCTGCAGAAACAGGGACGACTTTGTATATGCTTCTGT
TAGCGGCGTACAATGTACT
TTTACACAAATACTCGGGACAGGAAGAAATTGTAGTAGGAACGCCTATTGCCGGGCGATCT
CACGCAGATGTGGAAAATA
TTGTTGGGATGTTTGTCAATACGCTTGCATTGAAAAATACCCCTATAGCCGTACGCACCTT
CCACGAATTCCTGTTGGAA
GTAAAACAAAATGCTTTAGAAGCTTTTGAAAATCAAGACTATCCATTTGAAAATTTGATAG
AGAAGCTGCAAGTGCGTCG
CGACTTAAGTCGCAATCCATTATTTGATACAATGTTTAGCCTAAGCAATATTGACGAACAA
GTAGAGATAGGGATTGAGG
GATTGAACTTCAGCCCATATGAAATGCAGTATTGGATTGCAAAATTTGATATTTCATTCGA
TATTTTAGAAAAGCAAGAT
GACATTCAATTTTATTTTAACTATTGCACGAATCTGTTTAAAAAAGAAACGATAGAACGAT
TAGCGACACACTTTATGCA
TATTTTACAGGAGATTGTTATTAATCCTGAGATTAAGTTATGTGAAATTAATATGCTGTCC
GAAGAAGAACAGCAGCGTG
TCCTGTATGACTTTAATGGCACAGATGCAACCTACGCTACGAATAAAATATTCCATGAGTT
ATTTGAAGAACAGGTTGAA
AAAACACCAGATCATATAGCGGTGATAGATGAAAGAGAAAAGCTTTCCTATCAGGAGCTTA
ATGCGAAAGCGAATCAGCT
GGCACGAGTGCTGCGCCAAAAAGGAGTACAGCCTAATAGCATGGTAGGTATTATGGTAGAT
 
CGCTCACTCGACATGATTG
TAGGAATGCTTGGGGTTTTAAAAGCAGGAGGAGCATATGTGCCTATCGATATAGACTATCC
TCAGGAACGGATTAGCTAC
ATGATGGAAGATAGTGGTGCAGCGCTCTTGTTAACACAACAAAAGTTGACACAGCAAATTG
CGTTTTCTGGTGACATTTT
GTATCTTGACCAAGAAGAATGGCTTCATGAGGAAGCTTCAAATTTAGAACCCATCGCTCGT
CCGCAGGATATAGCCTATA
TCATTTACACTTCTGGTACAACCGGAAAGCCAAAAGGTGTGATGATTGAGCATCAAAGCTA
TGTGAATGTAGCAATGGCA
TGGAAAGATGCCTATCGGTTAGATACATTCCCGGTCCGTTTGCTTCAGATGGCTAGCTTTG
CCTTTGACGTATCTGCgGG
TGATTTTGCCAGAGCACTACTTACAGGTGGGCAATTAATTGTATGTCCAAATGAAGTAAAG
ATGGACCCAGCTTCTTTAT
ATGCCATTATTAAGAAATATGACATTACTATTTTTGAAGCAACGCCTGCTCTAGTGATTCC
ATTGATGGAGTATATTTAT
GAACAGAAGCTGGATATTAGCCAGTTACAGATTCTGATTGTCGGATCGGACAGTTGTTCGA
TGGAgGACTTTAAAACCTT
GGTTTCCCGTTTTGGTTCAACTATACGTATTGTGAATAGCTATGGAGTAACCGAAGCGTGC
ATTGATTCTAGCTATTATG
AACAACCGCTTTCTTCGTTACATGTAACAGGAACTGTACCGATTGGAAAACCGTACGCTAA
CATGAAAATGTATATTATG
AATCAATATTTGCAGATTCAGCCTGTAGGTGTAATTGGAGAATTATGTATTGGAGGAGCCG
GGGTTGCCCGTGGATATTT
AAATAGACCGGACTTAACAGCAGAAAAGTTTGTCCCTAATCCTTTTGTTCCAGGTGAAAAG
CTGTATCGAACAGGCGACT
TGGCAAGATGGATGCCGGATGGGAATGTTGAGTTTCTTGGTCGAAATGACCATCAGGTGAA
AATCAGAGGGATTCGAATC
GAGCTTGGAGAAATCGAAGCACAACTGCGTAAACATGATAGCATAAAAGAAGCAACTGTGA
TCGCAAGAGAAGATCACAT
GAAAGAGAAATATTTATGTGCGTATATGGTGACCGAAGGAGAAGTAAATGTAGCTGAACTG
CGTGCGTATCTAGCAAATG
ATCTGCCTGCGGCAATGATTCCGTCATATTTTGTATCGCTCGAAGCAATGCCACTTACTGC
TAATGGAAAAATTGATAAG
CGATCTTTACCAGAGCCCGATGGTTCCATATCGATAGGAACAGAATATGTAGCTCCGCGTA
CCATGCTTGAGGGAAAACT
AGAAGAGATATGGAAAGATGTATTGGGTTTACAGCGTGTTGGCATTCACGATGACTTCTTT
ACAATAGGTGGCCATTCAT
TGAAGGCTATGGCTGTTATTTCGCAAGTTCATAAAGAATGCCAGACTGAAGTTCCTCTGCG
TGTCTTATTTGAAACACCT
 
ACCATTCAAGGACTGGCTAAATATATAGAGGAaACGGACACAGAGCAATATATGGCTATTC
AGCCGGTTAGCGGACAGGA
CTATTATCCAGTATCATCAGCACAAAAGAGAATGTTTATTGTTAATCAATTTGATGGAGTA
GGAATTAGCTACAATATGC
CTTCCATCATGCTGATTGAAGGAAAACTTGAGCGAACACGCTTGGAATCAGCATTTAAAAG
ATTGATAGAACGACATGAG
AGCCTTCGAACATCTTTTGAAATAATAAATGGTAAGCCTGTACAGAAGATTCATGAGGAAG
TTGATTTCAATATGTCCTA
TCAGGTGGCTTCTAATGAACAAGTAGAGAAGATGATCGATGAGTTCATTCAGCCTTTCGAT
TTAAGTGTTGCACCGCTGC
TTCGTGTGGAACTTTTAAAATTGGAAGAgGACCGTCATGTGCTTATATTTGATATGCATCA
TATTATCTCAGATGGTATA
TCTTCCAATATTTTGATGAAAGAATTAGGAGAACTATATCAAGGTAATGCTTTACCAGAAC
TTCGTATTCAATACAAGGA
TTTCGCTGTATGGCAAAATGAGTGGTTCCAGTCAGAAGCCTTTAAAAAGCAAGAAGAATAC
TGGGTAAATGTtTTCGCAG
ATGAACGCCCGATTCTGGATATACCGACGGATTATCCAAGGCCGATGCAACAAAGCTTTGA
TGGTGCTCAACTTACATTT
GGAACCGGAAAGCAGCTTATGGATGGGTTATACAGGGTAGCAACGGAAACGGGAACAACGC
TTTATATGGTTTTGCTTGC
GGCATATAATGTTCTTCTTTCCAAATATTCTGGTCAAGAAGATATTATTGTAGGGACACCG
ATTGTGGGTAGATCCCATA
CTGACCTTGAGAATATTGTCGGGATGTTTGTCAACACGTTAGCAATGAGAAATAAACCGGA
AGGAGAAAAGACGTTCAAA
GCATTTGTATCAGAAATAAAGCAGAATGCACTAGCGGCTTTTGAGAATCAGGATTATCCAT
TTGAgGAGCTTATCGAAAA
ACTAGAGATACAAAGGGACTTAAGCAGAAATCCATTATTTGATACGCTCTTTAGCCTTCAA
AACATAGGTGAAGAATCAT
TTGAACTAGCCGAATTAACATGCAAACCTTTCGATTTGGTAAGCAAATTAGAGCATGCCAA
GTTTGATCTGAGTCTTGTG
GCAGTAGAAAAAGAGGAAGAAATTGCATTTGGGCTTCAATACTGCACAAAACTGTATAAGG
AAAAAACAGTTGAACAACT
GGCTCAACATTTTATTCAAATAGTAAAAGCAATTGTAGAAAATCCAGATGTCAAATTATCT
GATATTGATATGTTATCTG
AAGAAGAGAAGAAACAAATCATGCTTGAGTTCAATGATACGAAAATACAATATCCGCAGAA
TCAAACAATACAGGAATTG
TTTGAgGAGCAAGTGAAGAAAACACCTGAACATATAGCAATCGTATGGGAAGGGCAAGCAT
TAACCTATCATGAGCTAAA
TATAAAAGCTAATCAGTTAGCTCGTGTATTACGAGAAAAAGGGGTAACCCCTAATCATCCT
 
GTAGCGATTATGACGGAAC
GCTCATTAGAGATGATCGTAGGTATCTTTAGTATTTTGAAAGCAGGAGGAGCATATGTTCC
AATTGATCCAGCCTATCCA
CAAGAACGTATTCAATACTTGCTTGAAGATAGCGGAGCGACGCTACTGCTTACTCAGTCAC
ATGTATTAAATAAATTACC
GGTCGATATCGAATGGTTGGATCTTACAGATGAACAAAACTATGTAGAAGATGGTACCAAT
CTTCCATTTATGAATCAGT
CAACAGATCTTGCCTATATTATTTATACATCCGGTACAACAGGCAAGCCTAAAGGGGTTAT
GATTGAACATCAAAGCATC
ATCAACTGCCTGCAATGGCGGAAGGAAGAATACGAATTTGGACCAGGGGATACGGCTCTAC
AAGTGTTTTCCTTTGCTTT
TGATGGATTTGTAGCAAGTTTGTTTGCTCCGATTCTTGCtGGTGCAACGTCTGTTCTCCCT
AAGGAGGAAGAAGCAAAAG
ATCCAGTTGCATTGAAAAAACTGATCGCATCAGAAGAGATTACACATTACTACGGTGTGCC
TAGTTTGTTTAGTGCCATT
CTTGATGTTTCTTCTAGTAAGGATTTGCAAAATTTACGCTGCGTCACTTTGGGAGGAGAGA
AATTACCGGCTCAAATTGT
TAAAAAAATCAAAGAAAAAAATAAAGAAATTGAAGTCAACAACGAATATGGGCCTACTGAA
AATAGTGTAGTAACTACTA
TTATGCGCGATATACAGGTAGAACAAGAGATTACTATTGGTCGCCCATTATCTAACGTAGA
TGTATATATTGTCAATTGT
AATCATCAATTACAACCAGTAGGTGTAGTAGGGGAATTATGTATTGGTGGACAGGGACTTG
CAAGAGGATATTTGAATAA
ACCAGAGCTTACAGCAGATAAATTTGTTGTAAATCCATTCGTACCTGGTGAACGTATGTAC
AAAACCGGTGACCTTGCAA
AATGGCGCTCAGATGGAATGATTGAATATGTGGGGCGTGTTGATGAACAAGTAAAAGTAAG
AGGATATCGGATTGAGCTT
GGTGAAATTGAATCAGCTATCCTAGAATACGAAAAAATTAAGGAAGCGGTAGTTATGGTTT
CGGAGCATACTGCATCTGA
ACAGATGTTATGTGCTTATATTGTAGGGGAAGAAGATGTACTGACTCTGGACTTAAGAAGC
TATCTAGCAAAATTACTAC
CAAGTTATATGATTCCAAACTATTTTATCCAATTGGATAGTATTCCGCTTACACCAAACGG
TAAAGTGGATCGTAAAGCA
TTGCCTGAACCTCAAACCATTGGCTTAATGGCAAGGGAGTATGTTGCACCAAGGAATGAAA
TCGAAGCACAGCTAGTACT
CATTTGGCAAGAGGTATTAGGAATAGAACTGATCGGTATTACCGATAATTTCTTTGAATTA
GGAGGGCATTCTTTAAAGG
CAACGCTTTTAGTTGCAAAAATTTACGAGTACATGCAAATAGAGATGCCATTAAATGTTGT
GTTTAAACATTCAACTATT
 
ATGAAAATAGCGGAATATATTACACATCAAGAATCAGAAAATAATGTACATCAGCCTATTT
TGGTAAATGTAGAAGCAGA
TAGAGAGGCGCTATCTCTTAACGGCGAGAAGCAAAGAAAAAATATAGAGCTACCTATTCTG
CTAAACGAAGAAACAGATC
GAAACGTATTCTGCTTCGCGCCCATTGGTGCACAAGGTGTTTTTTATAAAAAGCTTGCTGA
ACAAATCCCTACTGCATCC
TTGTATGGCTTTGACTTCATTGAAGATGATGATCGAATTCAGCAATATATTGAATCGATGA
TTCAAACTCAGTCAGACGG
ACAATATGTGCTAATTGGTTATTCTTCAGGAGGGAACCTGGCTTTTGAAGTAGCAAAAGAA
ATGGAAAGGCAAGGATATA
GTGTATCTGATTTGGTCTTGTTCGATGTTTACTGGAAGGGAAAAGTaTTCGAGCAAACAAA
AGAAGAAGAAGAAAAC
ATAAAAATAATAATGGAAGAATTAAGGGAAAATCCAGGAATGTTCAATATGACACGAGAGG
ATTTTGAACTGTATTTTGC
GAATGAATTTGTGAAACAAAGTTTCACACGGAAAATGCGCAAATACATGAGTTTTTATACG
CAGTTAGTTAATTATGGGG
AAGTAGAAGCTACAATTCACCTTATACAAGCAGAATTTGAGGAAGAAAAAATTGACGAAAA
CGAAAAAGCCGACGAAGAA
GAAAAAACATATCTAGAGGAAAAATGGAATGAAAAAGCATGGAACAAAGCAGCAAAAAGAT
TTGTAAAATATAACGGATA
TGGCGCTCATTCTAACATGCTAGGAGGTGATGGTTTAGAGAGAAATTCCTCTATCCTTAAA
CAGATACTACAAGGGACAT
TTGTAGTAAAATAAaagaagaagtgtgaaaaagcgcagctgaaatagctgcgcttttttgt
gtcataa
SEQ ID NO: 4: BBaK_780000-PabrB 
AAGAAGAAGTGTGAAAAAGCGCAGCTGAAATAGCTGCGCTTTTTTGTGTCATAA
TCCTAATATATACATACGCCCTGAAA
AAGAATAATTAAAATTAAAAATCTTTAATGAAAATAAAAAAAGTCTGTTCGTGTCGAATTT
TGTCGTATATAATCAAATG
AAATAAAAAACTTTGGTTCGAAAGTCTTGATTTAAAAGGATTTTTAGTAGGATAATAGCTT
TATTAAATATTTATAAAAT
GCTGTTATTTCGGTAGTTTCCAAGACATTACTGACTATAAGAACTAATTCTTACAATCAAT
AGTAAACAAAATGATTGAC GATTATTGGAAACCTTGTTATGCTATGAAGGTAAGGAT
SEQ ID NO: 5: BBaK _780000-PvalS 
AAGAAGAAGTGTGAAAAAGCGCAGCTGAAATAGCTGCGCTTTTTTGTGTCATAA
TCCTAGATTAGGATTGCGAGCAGCAA
AACATCAAAGGTTGTCGGAAAGAAAATGGTTCTGATTCCTTGAAAAATCGTAATGGGAATA
ATCACTTGTGCGGATACAA
GCCGAATTTGCCTTACCCATGGCGGCAGCGAATAAGAGCTGAAACGTTTCTTCACGGTGCA
 
TCCTCCTCTACGCATTTAC
AAGCATCATATGTAAACTTGGGCAATAGGTGCCTGCCCATCTGTATAGAAAGCGGTGTTTT
TGGAAAAGATGATTGACGA ATCAAAAACACTTTTACTATAATAAAGAACATAAATAA
SEQ ID NO: 6: BBaK_780000-Phag 
AAGAAGAAGTGTGAAAAAGCGCAGCTGAAATAGCTGCGCTTTTTTGTGTCATAA
TCCTTGAAGTAAAAGTGATTGCGGTT
GAAGGGGATCAAGTGAAGCTTGGAATTGACGCCCCAAAGCATATTGATATTCACAGGAAAG
AAATTTACTTGACCATTCA
GGAAGAAAATAACCGTGCAGCAGCGTTATCCAGCGATGTGATCTCCGCATTATCCTCACAA
AAAAAGTGAGGATTTTTTT
ATTTTTGTATTAACAAAATCAGAGACAATCCGATATTAATGATGTAGCCGGGAGGAGGCGC
AAAAGACTCAGCCAGTTAC AAAATAAGGGCACAAGGACGTGCCTTAACAACATATTC
SEQ ID NO: 7: BBaK_780000-PspoVG 
AAGAAGAAGTGTGAAAAAGCGCAGCTGAAATAGCTGCGCTTTTTTGTGTCATAA
TCCTTGCGGAAGTAAACGAAGTGTAC
GGACAATATTTTGACACTCACAAACCGGCGAGATCTTGTGTTGAAGTCGCGAGACTCCCGA
AGGATGCGTTAGTCGAGAT
CGAAGTTATTGCACTGGTGAAATAATAAGAAAAGTGATTCTGGGAGAGCCGGGATCACTTT
TTTATTTACCTTATGCCCG
AAATGAAAGCTTTATGACCTAATTGTGTAACTATATCCTATTTTTTCAAAAAATATTTTAA
AAACGAGCAGGATTTCAGA AAAAATCGTGGAATTGATACACTAATGCTTTTATATAG
SEQ ID NO: 8: BBaK_780000-PsrfAA 
AAGAAGAAGTGTGAAAAAGCGCAGCTGAAATAGCTGCGCTTTTTTGTGTCATAA
TCCTGACGCTCTTCGCAAGGGTGTCT
TTTTTTGCCTTTTTTTCGGTTTTTGCGCGGTACACATAGTCATGTAAAGATTGTAAATTGC
ATTCAGCAATAAAAAAAGA
TTGAACGCAGCAGTTTGGTTTAAAAATTTTTATTTTTCTGTAAATAATGTTTAGTGGAAAT
GATTGCGGCATCCCGCAAA
AAATATTGCTGTAAATAAACTGGAATCTTTCGGCATCCCGCATGAAACTTTTCACCCATTT
TTCGGTGATAAAAACATTT TTTTCATTTAAACTGAACGGTAGAAAGATAAAAAATAT
SEQ ID NO: 9: BBaK_780000-Pcdd 
AAGAAGAAGTGTGAAAAAGCGCAGCTGAAATAGCTGCGCTTTTTTGTGTCATAA
TCCTTATTGAGTGGATGATTATATTC
CTTTTGATAGGTGGTATGTTTTCGCTTGAACTTTTAAATACAGCCATTGAACATACGGTTG
ATTTAATAACTGACAAACA
TCACCCTCTTGCTAAAGCGGCCAAGGACGCTGCCGCCGGGGCTGTTTGCGTTTTTGCCGTG
ATTTCGTGTATCATTGGTT
TACTTATTTTTTTGCCAAAGCTGTAATGGCTGAAAATTCTTACATTTATTTTACATTTTTA
GAAATGGGCGTGAAAAAAA GCGCGCGATTATGTAAAATATAAAGTGATAGCGGTACC
SEQ ID NO: 10: BBaK_780000-PlytR 
AAGAAGAAGTGTGAAAAAGCGCAGCTGAAATAGCTGCGCTTTTTTGTGTCATAA
TCCTCTAACCCTACATAAGTACCTTC
TTTTGTTTCAATGTTACTGTCTGGCGATACATCTTCACCTTGACTCTTTTGACTATTAACC
CCGCAACCCGAAAGAAGCA
ATATAAAGAACAGTAAAGCAATAAATTTTTTCATTTTTTTCACCTCATTATATTTTATCGT
CAACCTATTTTATATTTTA
AAGAAAAATTAAGAAACAATGAAACTTTTTTTTATAAAAAACGACTATTTTAGGATTTCAT
TCTTGTATTAAATAGAGTT GTATTTATTGGAAATTTAACTCATAATGAAAGTAATTT
SEQ ID NO: 11: BBaK_780000-Pveg 
AAGAAGAAGTGTGAAAAAGCGCAGCTGAAATAGCTGCGCTTTTTTGTGTCATAA
TCCTTATGGGAAGTGCTCCGTAATAC
GCTGACAAGAGAGAAAGGGCTTGGAGGTATTGAAACAAGAGGAGTTCTGAGAATTGGTATG
CCTTATAAGTCCAATTAAC
AGTTGAAAACCTGCATAGGAGAGCTATGCGGTTTTTATTTTACATAATGATACATAATT
TACCGAAACTTGCGGAACA
TAATTGAGGAATCATAGAATTTTGTCAAAATAATTTTATTGACAACGTCTTATTAACGTTG
ATATAATTTAAATTTTATT TGACAAAAATGGGCTCGTGTTGTACAATAAATGTAGTG
SEQ ID NO: 12: BBaK_780000-PmmgA 
AAGAAGAAGTGTGAAAAAGCGCAGCTGAAATAGCTGCGCTTTTTTGTGTCATAA
TCCTTCGATATATCGCGTCTATTCCG
GCTTCCGGCTATCACCCGAAGATAAACAGCCCAGGGGTCACAGATGAAGTACTGAAGAAAA
TGAGGAACGGTTTGATTAA
GGTAAGGCCGTATACAGTCAATCGTCCGGAAGATATGAAGCGTCTCATTGAAGCGGGAGCA
GACGGCATGTTTACCGACT
TTCCAGAAAAGGCTTCGGCATTGCTGAAAAATGAATAGTTGTTAGAAGGAGGCTGTTTGAC
GCAGCCTTCTTTTTTCATT CATTCATGCCCGTTTCAAAGCATACATTCATAGAAGAC
SEQ ID NO: 13: BBaK_780000-PyqfD 
AAGAAGAAGTGTGAAAAAGCGCAGCTGAAATAGCTGCGCTTTTTTGTGTCATAA
TCCTAGATGCCCCTGCACCTATCCCT
AACCGTATGGAACAGGCAAGACGGGAAGCGGAAGAAAGACGCAGGGAAACAGCAAGAAACC
TGAAAGGGCTGGAACGAGA
TCTTGCTGCTGCCAAACAAAAAACAGTATACACAAAACAAAAAATGCTTCAGGTGAATAAA
GACACCGTCGTACAGGGGA
TCGTTCTAGGAGAGGTGTTCGGACCTCCACGGGCGAAAAAACCTCACCGTACGATGCGCCC
GGCCCGTAAAAATTAAAGT GTTAGAACCTCCTTTCAAATCATACATATGAGATGAAA
SEQ ID NO: 14: BBaK_780000-PsigW

AAGAAGAAGTGTGAAAAAGCGCAGCTGAAATAGCTGCGCTTTTTTGTGTCATAA
TCCTTGCCCCCCTCCACCATTATTGG

GCTATAGCCAAGCGGTAAGGCAACGGACTTTGACTCCGTCATGCGTTGGTTCGAATCCAGC
TAGCCCAGTCACAGACACC
TTTGATCAAAAGGTGTCTTTTTTCTTTTCGGAAAAATCATTCCAACTTCTAACTGTTCAGT
CTGTATAATAATTTTAAAA
ATATGTTAAGGTAGTTTATTCACGAATTACCATCTACACCCTGCCAAAAATTTGATAAACT
TATTTTATAAAAAAATTGA AACCTTTTGAAACGAAGCTCGTATACATACAGACCGGT

## Claims

1. A method for producing a strain having a desired property, the method comprising:
a step 1) of providing a construct comprising a gene cluster and a plurality of promoters;
a step 2) of performing combinatorial library construction for the promoters in the construct; and
a step 3) of selecting, in a host cell, a strain having a desired property.

2. The method according to claim 1, wherein the plurality of promoters are arranged in a monocistronic configuration for each of the genes contained in the gene cluster, are arranged in a polycistronic configuration for all of the genes in the gene cluster, or are arranged in a mixture of a monocistronic configuration and a polycistronic configuration with respect to the genes in the gene cluster.

3. The method according to claim 1, wherein the strain having the desired property has at least one property selected from the following properties: a property of being able to produce a target substance; a property of highly producing a target substance; a property of retaining a target substance within the cells without secreting the target substance; a property of secreting a target substance extracellularly; a property of enabling easy culture accompanied by production of a target substance; a property of being able to produce a plurality of target substances in a desired ratio; a property of producing a target substance without producing harmful substances; a property of having robust production of a target substance; a property in which no deletion occurs in a gene for producing a target substance during a culture period; a property of enabling control of production of a target substance; a property of being able to suppress production of substances other than a target substance; a property of being able to suppress production of by-products and/or impurities that are difficult to separate by affinity, ion exchange, gel filtration, reversed phase, normal phase, chiral chromatography, distillation, or crystallization; a property of producing a small amount of empty capsids in viral vector production; and a property in which no deletion occurs in a plasmid.

4. A method comprising performing an action based on the desired property by using a strain produced by the method according to claim 1.

5. The method according to claim 1, further comprising a step 4) of subjecting the construct of a single strain or a plurality of strains selected in the step 3) to combinatorial library construction and introducing the construct into a target host cell; and 5) a step of performing the step 3) again.

6. A method for producing a substance in a host organism, the method comprising:
a step (A) of constructing a construct in which a promoter introduction site and a sequence for reconstruction are added to a gene cluster involved in production of the substance having undesired restriction enzyme recognition sequences removed;
a step (B) of introducing, into a host organism for combinatorial library construction, a plurality of types of constructs in which promoters are introduced into the promoter introduction sites;
a step (C) of performing combinatorial library construction of promoter regions among the plurality of types of constructs by a Combi-OGAB^{™} method;
a step (D) of culturing a host organism for production having a construct forming a combinatorial library and selecting a plurality of strains based on any criterion;
a step (E) of performing combinatorial library construction again for the promoter regions in the constructs of the plurality of strains by a Combi-OGAB^{™} method; and
a step (F) of producing a substance by using a strain having a desired property.

7. The method according to claim 6, wherein the host organism for combinatorial library construction and the host organism for production are the same.

8. The method according to claim 6, wherein the host organism for combinatorial library construction and the host organism for production are different.

9. The method according to claim 7, wherein the host organism is Bacillus subtilis.

10. A method for producing a substance in a host organism, the method comprising a step of producing the substance by using a strain produced by the method according to claim 1.

11. A method for producing a substance in a host organism, the method comprising a step of producing the substance by using a strain obtained through the steps (A) to (E) in the method according to claim 6.

12. The method according to any one of claims 6 to 11, further comprising modifying the strain.

13. The method according to claim 6, further comprising a step of repeating the steps (D) and (E) multiple times.

14. The method according to claim 6, wherein the construction of the construct in the step (A) is performed by an OGAB^{™} method.

15. The method according to claim 6, wherein the promoters are a plurality of promoters different from one another in transcription initiation timing and/or transcription strength.

16. The method according to claim 6, wherein the host organism for production is Bacillus subtilis, actinomycetes, filamentous fungi, yeast, hydrogen-oxidizing bacteria, coryneform bacteria, or animal cells.

17. The method according to claim 6, wherein the host organism is Bacillus subtilis.

18. The method according to claim 6, wherein the undesired restriction enzyme recognition sequences are Type IIS restriction enzyme recognition sequences selected from the group consisting of AarI, BsaI, BbsI, BsmBI, and BspQI, and an SfiI recognition sequence for reconstruction.

19. The method according to claim 6, wherein the sequence for reconstruction is an SfiI recognition sequence.

20. The method according to claim 6, wherein the gene cluster is a gene cluster of polyketide synthases (PKSs) and non-ribosomal peptide synthases (NRPSs).
